(19) Europäisches Patentamt
European Patent Office
Office européen des brevets

(11) Veröffentlichungsnummer: **0 227 047**
**A2**

# (12) EUROPÄISCHE PATENTANMELDUNG

(21) Anmeldenummer: **86117657.6**

(22) Anmeldetag: **18.12.86**

(51) Int. Cl.[4]: **C07D 213/65**, C07D 213/69, C07D 213/73, C07D 213/89, C07D 213/64, C07D 213/77

(30) Priorität: **21.12.85 DE 3545570**

(43) Veröffentlichungstag der Anmeldung:
**01.07.87 Patentblatt 87/27**

(84) Benannte Vertragsstaaten:
**AT BE CH DE ES FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Fuss, Andreas, Dr.**
**Lindigstrasse 24**
**D-8757 Karlstein(DE)**
Erfinder: **Koch, Volker, Dr.**
**Altkönigstrasse 5**
**D-6233 Kelkheim(DE)**

## (54) Neue Pyridin-Derivate und deren N-Oxide, Verfahren zu ihrer Herstellung und ihre Verwendung als Zwischenprodukte.

(57) Die Verbindungen der Formel I

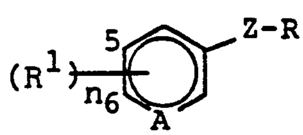

(I),

worin A = N oder N → O, Z = O oder NH, R = H, (Halo)Alkyl, (Halo)Alkenyl, (Halo)Alkinyl oder Alkoxycarbonyl, R' = Wasserstoff, Halogen, Amino, -NHOH, Hydroxy, (subst.)Phenylazo oder einen Rest der Formeln Y = C = N-, Y'Y²C = N-, Y³NH-oder

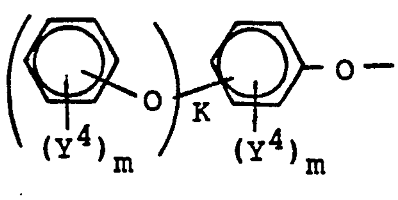

K = 0 oder 1, m,n = unabhängig voneinander eine Zahl von 1 bis 4 bedeuten, mit der Maßgabe, daß, wenn Z-R = OH oder $NH_2$ bedeutet, $(R')_n$ = mindestens einen Rest der Formel

EP 0 227 047 A2

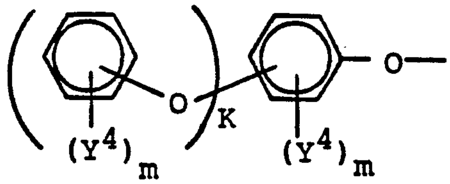

bedeutet oder für zwei Reste in 5-oder 6-Position des heterocyclischen Ringes steht, die in Position 5 Halogen und in Position 6 ein Rest der Gruppe Halogen, Amino, Hydroxy oder Phenylazo, das wie oben angegeben substituiert sein kann, bedeuten, sind wertvolle Zwischenprodukte bei der Synthese von Pflanzenschutzmitteln.

**Neue Pyridin-Derivate und deren N-Oxide, Verfahren zu ihrer Herstellung und ihre Verwendung als Zwischenprodukte**

Hydroxy-und Aminopyridin-Derivate sind aus K.M. Dynmaer and L.D. Smirnov, Russ.Chem.Rev. 44, 823 (1975), A.S. Tomcufcik and L.N. Starker in E. Klinsberg,The Chemistry of Heterocyclic Compounds, Band 14, S. 1-155, Interscience Publishers, Wiley, New York 1962 bekannt.

Gegenstand der vorliegenden Erfindung sind die neuen Verbindungen der Formel I

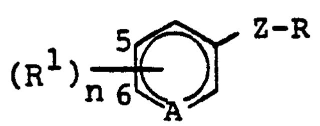

(I),

worin

A= N oder N → O,

Z O oder NH,

R H, halogeniertes $(C_1-C_8)$Alkyl, halogeniertes $(C_2-C_8)$Alkenyl oder halogeniertes $(C_2-C_6)$Alkinyl, sowie für Z=O $(C_1-C_4)$Alkoxycarbonyl,

R' bei gleicher oder verschiedener Bedeutung Wasserstoff, Halogen, Amino, -NHOH, Hydroxy, Phenylazo, das im Phenylring ein-oder mehrfach durch Halogen, Nitro, Carboxy, Sulfo $(C_1-C_8)$-Alkyl, $(C_1-C_8)$-Alkoxy, halogeniertes $(C_1-C_8)$Alkyl oder halogeniertes $(C_1-C_8)$Alkoxy substituiert sein kann,

einen Rest der Formeln

$Y=C=N-$, $Y^1Y^2C=N-$, $Y^3NH$-oder

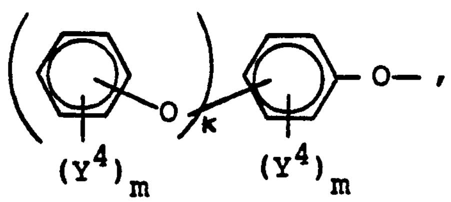

wobei für diesen Rest n vorzugsweise 1 bedeutet,

Y = O oder S

$Y^1$ = Halogen, $(C_1-C_3)$Alkylthio, $(C_1-C_3)$Alkylsulfenyl, $(C_1-C_3)$Alkylsulfonyl,

$Y^2$ = einen für $Y^1$ genannten Rest oder Mercapto,

$Y^3$ = $(C_1-C_6)$Alkyl, $(C_1-C_6)$Alkylsulfenyl, die beide halogeniert sein können, Phenylsulfenyl, das durch $(C_1-C_6)$-Alkyl, $(C_1-C_6)$Alkoxy $(C_1-C_6)$Alkoxy-carbonyl, Halo$(C_1-C_6)$alkyl, Halo$(C_1-C_6)$alkoxy, Halo$(C_1-C_6$alkyl)carbonyl, Halogen, Nitro oder Cyano substituiert sein kann, Phosphoryl oder Thiophosphoryl, die beide durch zwei Reste der Gruppe $(C_1-C_8)$Alkoxy, $(C_1-C_6)$Alkylthio, Amino, $(C_1-C_6)$Alkylamino oder Di$(C_1-C_6$alkyl)amino substituiert sein können,

$Y^4$ = unabhängig voneinander H, Halogen, $NO_2$, $NH_2$, $-NHY^3$, -NHOH, -NCO, -NCS, OH, $(C_1-C_6)$Alkyl, $(C_2-C_6)$-Alkenyl, $(C_2-C_6)$Alkinyl, $(C_1-C_6)$Alkoxy, $(C_1-C_6)$Alkoxy-carbonyl, wobei die fünf letzt genannten Reste halogeniert sein können, Nitro, Cyano, Carboxy oder ein Carboxylat-Salz,

K = 0 oder 1,

m,n = unabhängig voneinander eine Zahl von 1 bis 4 bedeuten, mit der Maßgabe, daß, wenn Z-R=OH oder $NH_2$ bedeutet, no ist und mindestens ein Rest R' = einen Rest der Formel

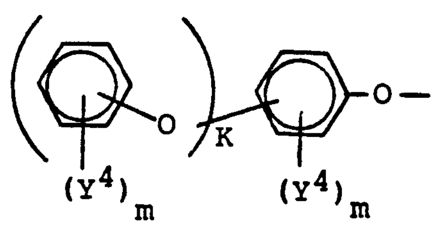

bedeutet oder $(R^1)_n$ für zwei Reste in 5-oder 6-Position des heterocyclischen Ringes steht, und zwar in Position 5 für Halogen und in Position 6 für einen Rest der Gruppe Halogen, Amino, Hydroxy oder Phenylazo, das wie oben angegeben, substituiert sein kann.

Für Halogen steht insbesondere F, Cl oder Br, im Falle $Y^4$ besonders bevorzugt Cl. Die Vorsilbe "Halo" bedeutet ein oder mehrere Halogenatome.

R bedeutet vorzugsweise H, $CF_2H$, $CF_3$, $CF_2CHF_2$, $CF_2CHFCl$, $CF_2CHFBr$, $CF_2CHFCF_2$, $CH_2CF_3$, $CFCl_2$ oder $CF_2Cl$.

Im Falle $R^1$= OH kann, insbesondere dann wenn $R^1$ in Position 2, 4 oder 6 steht die Verbindung der Formel I in Form der tautomeren Pyridone vorliegen.

Bevorzugt sind die Verbindungen der Formel I, worin A = N, N→O, Z = O oder NH,
R = H, halogeniertes $(C_1$-$C_8)$Alkyl,
$R^1$ bei gleicher oder verschiedener Bedeutung Wasserstoff, Halogen, Amino, Hydroxy, Phenylazo, das im Phenylring ein-oder mehrfach durch Halogen, Nitro oder Carboxy substituiert sein kann,
einen Rest der Formeln
Y = C = N-, $Y^1Y^2C$ = N-oder

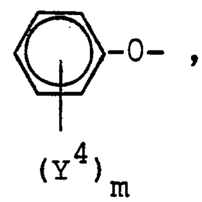

Y = O oder S
$Y^1$ = Halogen oder $(C_1$-$C_3)$Alkylthio,
$Y^2$ = einen für $Y^1$ genannten Rest oder Mercapto,
$Y^4$ = unabhängig voneinander H, $NO_2$, $NH_2$, -NHOH, Halogen, -NCO, -NCS, OH, $(C_1$-$C_6)$Alkyl, $(C_1$-$C_6)$Alkoxycarbonyl, wobei die zwei letzt genannten Reste halogeniert sein können,
m,n = unabhängig voneinander eine Zahl von 1 bis 4 bedeuten.

Besonders bevorzugt sind die Verbindungen der Formel I, worin
A = N
Z = O oder NH,
R = H oder halogeniertes $(C_1$-$C_4)$Alkyl,
$R^1$ = H, Halogen, Amino, Hydroxy oder

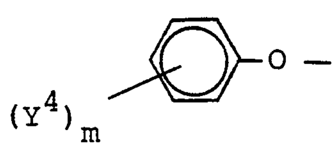

$Y^4$ = H, Cl, $CF_3$, $NH_2$, -NCO oder $(C_1$-$C_2)$Alkoxycarbonyl,
die in Position 2, 4 oder 6 des Phenoxyrestes orientiert sind, und
m,n = eine Zahl von 1 bis 3 bedeuten.

Gegenstand der vorliegenden Erfindung sind auch Verfahren zur Herstellung der Verbindungen der Formel I, dadurch gekennzeichnet, daß man

a) zur Herstellung von Verbindungen mit Z = O und R≠H eine Verbindung der Formel (II)

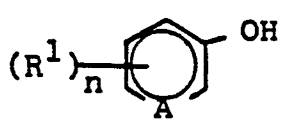
(II)

$a_1$) mit einer Verbindung der Formel $Y^1$-R', worin R' die Bedeutung von R außer H und $Y^1$ einen nucleofugen Rest wie Halogen, Phenylsulfonyl, Phenylsulfinyl, Tosyl oder Mesyl bedeutet, in Gegenwart einer Base oder

$a_2$) mit einem halogenierten ($C_2$-$C_8$)Alken oder halogeniertem ($C_2$-$C_8$)Alkin in Gegenwart einer Base, oder

$a_3$) einem halogeniertem Carben der Formel $C(Hal)_2$), wobei Hal bei gleicher oder verschiedener Bedeutung Halogen, insbesondere F oder Cl bedeutet, oder

$a_4$) mit einer Verbindung der Formel $Y^1$-CO-$R^2$, worin $R^2$ = Halogen, halogeniertes ($C_1$-$C_7$)Alkyl, halogeniertes ($C_2$-$C_7$)Alkenyl oder halogeniertes ($C_2$-$C_7$)Alkinyl bedeutet, umsetzt und das erhaltene Zwischenprodukt der Formel (III)

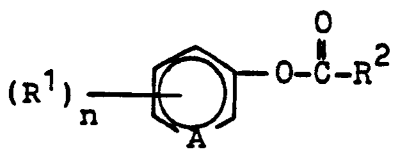

(III)

halogeniert, oder

$a_5$) zur Herstellung von Verbindungen mit Z = O und R = perhalogeniertes ($C_1$-$C_8$)Alkyl eine Verbindung der Formel (II) mit einem perhalogenierten ($C_1$-$C_8$)Alkan in Gegenwart einer Base oder in Gegenwart von HF, oder

b) zur Herstellung von Verbindungen der Formel I mit Z = O und R* eine Verbindung der Formel (IV)

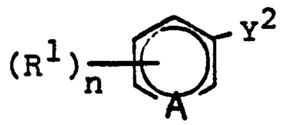

(IV),

worin $Y^2$ eine Abgangsgruppe wie Halogen, Nitro, Diazonium, Alkylsulfonyl, halogeniertes Alkylsulfonyl, Phenylsulfonyl, das im Phenylrest beispielsweise durch Alkyl, Halogen substituiert sein kann, bedeutet, mit einem Alkalisalz einer Verbindung der Formel OH-$CH_2$-$R^3$, worin $R^3$ die Bedeutung von $R^2$ außer Halogen besitzt, umsetzt oder

c) zur Herstellung von Verbindungen der Formel I mit Z = O und R = H eine Verbindung der Formel -(V),

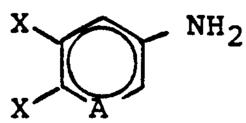

(V)

wobei X = unabhängig voneinander Halogen bedeutet, diazotiert und hydrolysiert oder

d) zur Herstellung von Verbindungen mit Z-R = $NH_2$ eine Verbindung der Formel (VI)

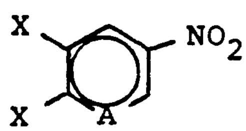

(VI)

reduziert, oder

e) zur Herstellung von Verbindungen der Formel I, die in Position 6 einen, gegebenenfalls wie oben angegeben substituiert Phenylazo-Rest enthalten, eine Verbindung der Formel (VII)

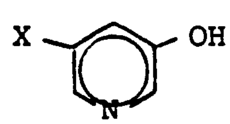

(VII)

mit einem Phenyldiazonium-Ion, das, wie für Phenylazo von Formel (I) angegeben, substituiert sein kann,

umsetzt, oder

f) zur Herstellung von Verbindungen mit

$R^1$=

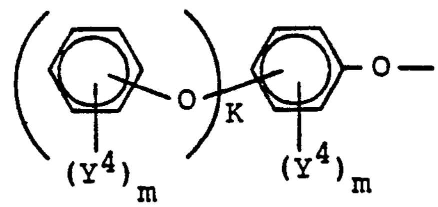

eine Verbindung der Formel (VIII)

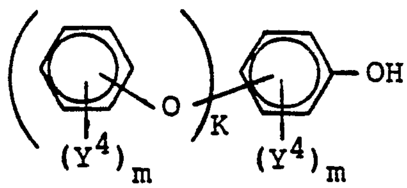

(VIII)

mit einer Verbindung der Formel IX

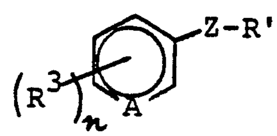

(IX)

worin $R^3$ eine nucleofuge Abgangsgruppe wie Halogen, Alkylsulfinyl, Phenylsulfinyl, Phenylsulfonyl oder Alkylsulfonyl bedeutet, und R' die Bedeutung von R außer Wasserstoff besitzt, umsetzt,
und die unter a) bis f) erhaltenen Produkte gegebenenfalls derivatisiert.

Die als Ausgangsverbindungen bei den unter a) genannten Herstellungsverfahren zu verwendenden Pyridinole der For mel II sind entweder bekannt, s. A. Weissberger u. E.C. Taylor, The Chemistry of Heterocyclic Compounds, Pyridine and its Derivatives, Wiley, New York, oder lassen sich gemäß Verfahren c) und e) bzw. nach dem Fachmann geläufigen Methoden herstellen.

Die als Ausgangsverbindungen in den Verfahren b),d) oder f) zu verwendenden Pyridine der Formeln IV, VI oder IX sind literaturbekannt oder lassen sich analog zu bekannten Verfahren herstellen, s. A. Weissberger, E.C. Taylor loc.cit. oder T. Batkowski, Rosz. Chem. 1968 (12) 2079.

Die als Ausgangsverbindungen beim Herstellungsverfahren e) zu verwendenden 3-Pyridinole der Formel VII sind literaturbekannt oder lass sich analog zu bekannten Verfahren herstellen, s. T. Batkowski loc.cit..

(II) kann gemäß Variante $a_1$) in der Weise alkyliert werden, daß man (II) mit einem Alkylierungsreagenz wie z.B. Methylchlorid, Methylbromid, Methyljodid, Dimethylsulfat, Diethylsulfat, p-Toluolsulfonsäuremethylester, Fluorsulfonsäuremethylester, Trifluormethansulfonsäuremethylester, Methansulfonsäureethylester, Fluorsulfonsäure2.2.2-trifluorethylester, Fluorsulfonsäure-1-hydroperfluorisopropylester in Gegenwart einer Base wie Alkalihydroxid oder Alkalicarbonat umsetzt. Als Lösungsmittel kann beispielsweise Aceton, Acetonitril, Dimethylformamid, Dichlormethan oder Dimethylsulfoxid eingesetzt werden, wahlweise in Gegenwart von Phasentransferkatalysatoren, wie TEBA oder Aliquat 336. Die Reaktion wird bei Temperaturen zwischen -100 und +200°C, vorzugsweise zwischen 0 und 100°C durchgeführt, s. J.March, Advanced Organic Chemistry Tokyo 1977, S. 357 ff. Die so erhaltenen Verbindungen werden dann gegebenenfalls in der Seitenkette halogeniert.

Bei der Verfahrensvariante $a_2$) entsteht eine Haloalkoxy-oder Haloalkenyloxy-Verbindung der Formel I. Diese kann durch Seitenkettenmodifikation beispielsweise durch Halogenwasserstoffaddition an eine Mehrfachbindung, beschrieben in Houben-Weyl-Müller Band V/3, Seite 811, ff, Georg-Thieme-Verlag, Stuttgart, 1962, durch Halogenaddition an eine Mehrfachbindung, beschrieben in Houben-Weyl-Müller, Band V/3 S. 529 ff, durch Austauch von Wasserstoff gegen Halogen, beschrieben in Houben-Weyl-Müller, Band V/3 S. 564 ff, oder durch Umhalogenierung beschrieben in Houben-Weyl-Müller, Band V/3 S. 749 ff 70 ff, Band

V/4, S. 354 ff, 595 ff, in andere Verbindungen der Formel I überführt werden. Als Base läßt sich bei Variante $a_2$) beispielsweise ein Alkalihydroxid, Alkalialkoholat, Alkalihydrid, Alkalicarbonat, Alkaliamid oder Trialkylamin einsetzen. Man arbeitet in einem dipolar aprotischen Lösungsmittel wie Tetrahydrofuran, Dioxan, Diglyme, Tetraglyme, Dimethoxyethan, Acetonitril, Propionitril, Benzonitril, Dimethylformamid, Dimethylacetamid, N-Methylpyrrolidon, Dimethylsulfoxid, Sulfolan, Dimethylsulfon, Diphenylsulfon, Aceton, Methylethylketon oder Hexamethylphosphorsäuretriamid bei Temperaturen von -100 bis +250°C, vorzugsweise -10 bis +100°C. Als Haloalken bzw. Haloalkin wird beispielsweise 1,1-Difluorethan, 1,2-Difluorethen, Trifluorethen, Tetrafluorethen, Chlortrifluorethen, Bromtrifluorethen, Jodtrifluorethen, 1,1-Dichlordifluorethen, Trichlorfluorethen, Tetrachlorethen, Hexafluorpropen, 1,1,1-Trifluorpropan, 2-Trifluormethyl-1,1,1-trifluor-2-propen,Octafluorisobuten, Perfluor-4-methyl-2-penten, Perfluor-4-methyl— 1-penten oder Perfluor-2-butin eingesetzt.

Das bei der Halocarbeninsertion gemäß Verfahrensvariante $a_3$) benutzte Dihalocarben der Formel $C(Hal)_2$ wird durch alpha-Elimination einer Trihalogenmethan-Verbindung wie z.B. Trichlormethan, Trifluormethan, Tribrommethan, Trijodmethan, Dichlorfluormethan, Dibromfluormethan, Bromdifluormethan, vorzugsweise Chlordifluormethan mit einer Base wie Alkalihydroxid, Alkalicarbonat, Alkalialkoholat oder Alkalihydrid in Lösungsmitteln, wie z.B. Dimethoxyethan, Tetrahydrofuran, Ethanol, Methanol, Dimethylformamid, N-Methylpyrrolidon, Dimethylsulfoxid, Aceton, vorzugsweise 1,4-Dioxan, Dimethylformamid oder Acetonitril bei Temperaturen von -100° bis +200°C, vorzugsweise von 0° bis 60°C erzeugt. Es können auch andere Dihalogencarbenquellen eingesetzt werden, wie Salze von Trihalogenessigsäuren, die thermisch zersetzt werden oder Trihalomethylmetallverbindungen, s. J. March, Advanced Organic Chemistry, S. 181, 343, 496, 789, Tokyo 1977.

Bei der Verfahrensvariante $a_4$) lassen sich als Reagenzien der Formel $Y^1$-CO-$R^2$ beispielsweise die Verbindungen Phosgen, Carbonylfluorid, Trichloracetylchlorid, Dichloracetylchlorid, Chloracetylchlorid, Trichloracetanhydrid, Dichloracetanhydrid, Perchlorpropionylchlorid, Dichlorfluoracetylanhydrid, Chlordifluoracetylchlorid, Trifluoracetylchlorid, Trifluoracetanhydrid, Perfluorpropionylchlorid oder Perfluoroctansäurechlorid einsetzen. Als Lösungsmittel werden verwendet beispielsweise Dichlormethan, Chloroform, Tetrachlormethan, Frigen 113, 1,2-Dichlorethan, Diethylether, Dimethoxyethan, Toluol, Benzol, Chlorbenzol, Xylol oder Kolenstoffdisulfid; es werden wahlweise organische Stickstoffbasen wie Triethylamin, Pyridin oder 4-Dimethylaminopyridin zugegeben. Die Reaktion kann zwischen -20° und +50°C durchgeführt werden.

Die Halogenierung der so erhaltenen Verbindungen der Formel I kann mit Schwefeltetrafluorid gegebenenfalls in Gegenwart von HF oder Phenylphosphonsäuredichlorid erfolgen, s. W.A. Sheppard, J.Org Chem. 29, (1964) und US-PS 4.419.514; CA 100 , 854 125.

Das Verfahren $a_5$) kann in der Weise durchgeführt werden, daß das Pyridinol der Formel II mit einem Perhalogenalkan wie z.B. Jodtrifluormethan, Bromtrifluormethan, Chlortrifluormethan, Dibromdifluormethan, Bromchlordifluormethan, Tetrachlormethan, 1,2-Dibromtetrafluorethan, 1,2-Dibromhexafluorpropan, Jodperfluorethan, 2-Jodperfluorpropan, 1-Jodperfluorhexan, 1-Jodperfluoroctan in Gegenwart einer Base wie Tetrabutylammoniumhydroxyd, Ammoniak, ein Alkalimetall, ein Alkalihydroxyd, ein Alkalihydrid oder ein Alkalicarbonat wahlweise unter Zusatz von einem Phasentransferkatalysator wie z.B. [18]Krone 6, [15]Krone 5, TEBA (Benzyltriethylammoniumchlorid) oder Aliquat 336 (Tricaprylmethylammoniumchlorid) in einem Lösungsmittel wie z.B. Benzol, Toluol, Dichlormethan, Dimethylformamid, N-Methylpyrrolidon, Sulfolan, Acetonitril, Tetrahydrofuran oder Tetraglyme, gegebenenfalls unter UV-Bestrahlung (180-350 nm), bei Temperaturen von -50°C bis +100°C und Drucken von 0 bis 100 bar umgesetzt wird. Als Alternative kann das Pyridinol (II) mit einem der obengenannten Perhalogenalkane in 500-5000 mol-% wasserfreier Flußsäure gegebenenfalls unter Zusatz von 0,1 -10 mol-% Bortrifluorid, Antimonpentachlorid oder Antimontrifluorid bei Temperaturen von 0 bis 180°C und Drucken von 0 bis 100 bar zu den Pyridinen der Formel I umgesetzt werden.

Weiterhin kann zur Alkylierung der Verbindungen der Formel I auch ein Verfahren angewendet werden, bei dem (II) zunächst in sein Anion überführt wird, anschließend ein Oxiran wie z.B. Hexafluorpropenoxid, Octafluorisobutenoxid, Perfluoriso-1-hexenoxid oder Perfluoriso-2-hexenoxid zugeführt wird und das erhaltene Carboxylat pyrolysiert wird, s. H.Millauer et al., Angew.Chem. 97, 164 (1985).

Das Verfahren gemäß Variante b) kann zwischen 0° und 200°C, vorzugsweise 80° und 170°C durchgeführt werden. Als Alkalisalze der Verbindungen der Formel $HO-CH_2R^3$ können beispielswese eingesetzt werden die Alkalisalze von 2.2.2-Trichlorethanol, 2.2.2-Trifluorethanol, 1-Hydroperfluorisopropanol, 2.2.3.3-Tetrafluorpropanol oder 2.2.3.4.4.4-Hexafluorbutanol. Als Lösungsmittel findet Anwendung beispielsweise Dimethoxyethan, Dimethylformamid, N-Methylpyrrolidon, Dimethylsulfoxid, Diemthylsulfon, Sulfolan, Adipodinitril oder Hexamethylphosphorsäuretriamid, vorzugsweise in Hexamethylphosphorsäuretriamid.

Bei der Umsetzung gemäß Variante c) wird das nach üblichen Methoden, s. Houben-Weyl, Bd X/3, S. 7 ff, erhaltene Diazoniumsalz von (V) entweder direkt in wäßriger Lösung erhitzt, s. Houben-Weyl, Bd VI/1c, S. 247 ff oder nach dessen Isolierung, z.B. als Tetrafluoroborat, s. Houben-Weyl, Bd X/3, S. 7 ff, hydrolysiert, oder zunächst acetyliert und anschließend hydrolysiert, s. Houben-Weyl, Bd VI/1c, S. 247 ff.

Die Hydrolyse erfolgt in wäßrig saurer Lösung bei Temperaturen zwischen +50°C und +150°C, bevorzugt zwischen +70°C und +120°C. Die Solvolyse in Eisessig oder Acetanhydrid kann bei Temperaturen von +20°C bis +140°C, bevorzugt zwischen +30°C und 100°C, erfolgen. Die Hydrolyse des so gebildeten Acetats kann bei Temperaturen von 0°C bis +100°C bevorzugt zwischen +20°C und 80°C in wäßrigem oder alkoholischem Alkali erfolgen.

Zur Derivatisierung der so erhaltenen Verbindungen der Formel I kann eine basenkatalytische Hydrolyse, Ammonolyse, Umsetzung mit Phenylhydrazinen mit anschließender Dehydrierung oder eine N-Oxidation unter Erhalt der N-oxide der Formel I, s. A. Weissberger und E.C. Taylor, The Chemistry of Heterocyclic Compopunds, Pyridine and its Derivatives, Wiley N.Y., durchgeführt werden.

Die Reduktion von VI im Verfahren d) kann mit einem anorganischen Reduktionsmittel (Houben-Weyl 4/1c, S. 563 ff und 4/1d S 1 ff) oder katalytisch erfolgen (Houben-Weyl, Band 4/1c S. 482 ff).

Als anorganisches Reduktionsmittels eignet sich beispielsweise Eisen, das keine Enthalogenierung bewirkt. Die Reaktion wird in wäßrigem Eisessig (5 bis 30-Gew.-%, bevorzugt 15-Gew.-%) durchgeführt. Die Reaktionstemperatur liegt im allgemeinen zwischen +10°C und +120°C, vorzugsweise zwischen +20 und +60°C.

Für die katalytische Hydrierung sind Metallkatalysatoren geeignet, die wenig aktiv sind, wie schwach aktives Raney-Nickel, Edelmetallsulfide, Osmium/Aluminiumoxid u.a. (Houben-Weyl, Band 4/1c, S. 520 ff). Als Lösungsmittel für die katalytische Hydrierung eignen sich niedere Alkohole, Glykol, Ether (z.B. Tetrahydrofuran, 1,4-Dioxan, Dimethoxyethan, 2-Methoxyethanol), Ester (z.B. Essigsäureester), Dimethylformamid, Pyridin und Eisessig. Die Zugabe von Basen und Salzen wie Magnesiumoxid, Calciumhydroxid, Natriumacetat sowie DMSO bewirkt ebenfalls eine Inhibierung der Enthalogenierung (Houben-Weyl, Band 4/1c, S. 521).

Bei der Umsetzung gemäß Variante e) wird das Pyridinol der Formel VII mit einer Phenyldiazonium-Verbindung vorzugsweise im pH-Bereich von 8 bis 10 in wäßrigem oder alkoholischem Medium bei Temperaturen von -20°C bis +10°C gekuppelt.

Darüberhinaus lassen sich bestimmte Verbindungen der Formel I durch Cyclisieren von Verbindungen des Typs

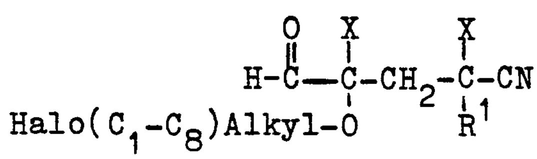

(X= Halogen)

mittels Kupfer oder Kupfersalzen in Gegenwart von Halogenwasserstoffen herstellen, vgl. EP-A 12 117.

Bei den oben angegebenen Verfahrensvarianten a) bis e) kann es zum Teil im Falle $R^1$= OH oder $NH_2$ erforderlich sein den Rest $R^1$ vor der Reaktion durch Einführung einer Schutzgruppe zu maskieren, um gute Ausbeuten zu erhalten, zur Anwendung von Schutzgruppen s. T.W. Greene, Protective Groups, Wiley 1981).

Die unter den Verfahren a) bis f) erhältlichen Verbindungen der Formel I können durch bekannte Reaktionsschritte in andere Verbindungen der Formel I überführt werden, beispielsweise durch Nitrierung, Alkylierung an einer Amino-oder OH-Gruppe, Sulfenylierung (vgl. Houben-Weyl, E11 S.110ff Phosphorylierung, (vgl. Houben-Weyl E2, S. 487ff), Reduktion einer Nitrogruppe zur Amino oder -NHOH-Gruppe, Einführung einer Aminogruppe durch Austausch von Halogen oder einer anderen Abgangsgruppe gegen Amino (vgl. Houben-Weyl, 11/1, S. 24ff), Phosgenierung einer Aminogruppe, Umwandlung einer Aminogruppe in die Gruppe S=C=N-auf üblichen Wegen (vgl. Houben-Weyl, E4, S. 738ff bzw. S. 834ff) oder Oxidation des Pyridins zum Pyridin-N-oxid (A. Weissberger und E.C. Taylor, loc.cit.).

Die Verbindungen der Formel I stellen wertvolle Zwischenprodukte bei der Herstellung von Pflanzenschutzmitteln dar, s. Deutsche Patentanmeldung P 35 45 569.1, "Neue Pyridin-Derivate, Verfahren zu ihrer Herstellung, sie enthaltende Mittel und ihre Verwendung als Schädlingsbekämpfungsmittel (HOE 85/F 294) und P 35 45 571.3, "Heterocyclische Phenyläther, Verfahren zu ihrer Herstellung und diese enthaltende Mittel (HOE 85/F 296).

Sie eignen sich ferner im Falle $R^1$ = substituiertes Phenylazo als Farbstoffe, vgl. G. Schiemann, B. Cornils, Chemie und Technologie cyclischer Fluorverbindungen, Ferdinand Enke-Verlag, Stuttgart (1969), S. 287-299.

Außerdem besitzen die Verbindungen der Formel (I) oberflächenaktive Wirkungen und eignen sich als Imprägnierungsmittel vgl. US-Patentschrift Nr. 2 492 855.

Die Erfindung wird durch nachfolgende Beispiele erläutert.

Chemische Beispiele

Beispiel 1

3-(2-Chlor-1,1,2-trifluorethoxy)-5,6-dichlorpyridin

In eine Mischung aus 16,4 g (0,1 Mol) 5,6-Dichlor-3-pyridinol I/29 und 20 g (0,145 Mol) wasserfreiem Kaliumcarbonat in 100 ml absolutem Acetonitril wurde bei 35°C Chlortrifluorethylen eingeleitet bis kein Ausgangsmaterial mehr vorhanden war. Der Feststoff wurde abgesaugt, das Lösungsmittel abdestilliert, der Rückstand in Ether aufgenommen und neutral gewaschen. Nach Trocknen über $MgSO_4$ und Abdestillieren des Ethers wurde im Hochvakuum destilliert.
Ausbeute 83 %
Kp 85°C/ 0,03 mbar

Beispiel 2

3-(1,1,2,3,3,3-Hexafluor-1-propyloxy)-2-hydroxypyridin

2,6 g (10 mmol) 2-Amino-3-(1,1,2,3,3,3-hexafluor-1-propyloxy) pyridin wurde in 35 ml 2n Schwefelsäure mit 0,76 g (11 mmol) Natriumnitrit in 5 ml Wasser bei 0°C diazotiert. Man rührte 30 Minuten nach und extrahierte das Produkt mit Essigester. Die organische Phase wurde neutral gewaschen, mit $MgSO_2$ getrocknet und eingedampft.
Ausbeute 75 %
Fp 89°C

Beispiel 3

5,6-Dichlor-3-difluormethoxypyridin

In ein Gemisch aus 32,8 g (0,2 Mol) 5,6-Dichlor-3-pyridinol in 200 ml 1,4-Dioxan und 40 g (1 Mol) Natriumhydroxid in 100 ml Wasser wurde bei 50°C solange Chlordifluormethan eingeleitet bis kein Ausgangsmaterial mehr vorhanden war. Dann wurde,wie in Beispiel 1 beschrieben,weiterverfahren.
Ausbeute 77 %
Kp 57 -60°C / 0,1 mbar

Beispiel 4

2-Chlor-5-(1.1.2.2-tetrafluorethoxy)-pyridin

Man versetzte ein Gemisch aus 13.6 g (65 mmol) 5-(1.1.2.2-Tetrafluorethoxy)-2-hydroxypyridin und 5.6 g (43 mmol) Chinolin mit 14.9 g (97 mmol) Phosphoroxychlorid und rührte 3 h bei 120°C. Die Mischung wurde bei 20°C auf Eis gegossen, mit Dichlormethan extrahiert und über Natriumsulfat getrocknet. Man dampfte das Solvens am Rotationsverdampfer ab und destillierte den Rückstand bei 6 mbar.
Ausbeute 11,7 g (79 %)
Kp 79°C / 6 mbar

Beispiel 5

2-Chlor-5-(2-chlor-1.1.2-trifluorethoxy)-pyridin

Zu eine Lösung von 22.6 g (0.1 mol) 2-Amino-5-(2-chlor-1.1.2-trifluorethoxy)-pyridin in 100 ml konz. HCl wurde bei 0°C unter Rühren eine Lösung aus 7.59 g (0.11 mol) Natriumnitrit in 15 ml Wasser zugetropft. Man rührte 1 h bei 50°C, stellte den pH-Wert mit 2N KOH auf 6.5 ein und extrahierte dreimal mit je 100 ml Dichlormethan. Nach Trocknen über Natriumsulfat und Abdampfen des Solvens am Rotationsverdampfer destillierte man bei 6.5 mbar und erhielt 13 g 2-Chlor-5-(2-chlor-1.1.2-trifluorethoxy)-pyridin als farblose Flüssigkeit.
Ausbeute 53 %
Kp 101°C / 6.5 mbar

Beispiel 6

5-(1.1.2.2-Tetrafluorethoxy)-2-hydroxypyridin

Zu einer Lösung aus 23 g (109 mmol) 2-Amino-5-(1.1.2.2-tetrafluorethoxy)-pyridin in 103ml Wasser und 21.9 g konz. Schwefelsäure wurde bei 0°C unter Rühren eine Lösung aus 10.3 g (149mmol) Natriumnitrit in 16.5 ml Wasser zugetropft. Man rührte 3 h bei 20 -25°C, saugte den farblosen Feststoff ab und wusch mit Wasser säurefrei.
Ausbeute 16.4 g (71 %)
Fp 103°C

Beispiel 7

2-Amino-5-(2-chlor-1.1.2-trifluorethoxy)-pyridin

In einem 1 l VA-Autoklaven wurden 85.1 g (o,24 mol) 5-(2-chlor-1.1.2-trifluorethoxy)-2-(4-nitrophenylazo)-pyridin in 600 ml Essigsäure unter Zusatz von 10 g Palladium (10%) auf Aktivkohle mit 61 bar (Aufnahme) Wasserstoff bei 20 -25°C hydriert. Man filtrierte und destillierte das Solvens am Rotationsdampfer ab. Der Rückstand wurde in 500 ml Dichlormethan aufgenommen und mit 100 ml 20%igem wäßrigen Natriumcarbonat und viermal mit je 100 ml Wasser gewaschen und über Natriumsulfat getrocknet. Anschließend dampfte man das Solvens am Rotationsverdampfer ab und destillierte den Rückstand im Hockvakuum.
Ausbeute 54 %
Fp 63°C, perlmuttartig schimmernde Blättchen

Beispiel 8

3-Chlor-5-(2-chlor-1.1.2.-trifluorethoxy)-2-hydroxypyridin

a) Man löste 23.6 g (104 mmol) 5-(2-Chlor-1,1,2-trifluorethoxy)-2-hydroxypyridin in 80 ml konz. HCl und tropfte unter Rühren eine Lösung aus 5.3 g (42 mmol) Kaliumchlorat in 60 ml Wasser zu. Man rührte 1 h bei 50 -60°C, filtrierte bei 0°C ab und wusch mit Eiswasser säurefrei. Nach Kristallisation aus Methanol/Wasser erhielt man 20.3 g farblose Kristalle.
Ausbeute 75 %
Fp 140°C

b) In einer gasdichten Rührapparatur wurden auf eine Mischung aus 11.1 g (o.1 mol) 2,5-Dihydroxypyridin 13.8 g (0,1 mol) Kaliumcarbonat und 100 ml trockenem Dimethylformamid unter Rühren bei 75°C 2,2 l Chlortrifluorethylen aus einer Gasbürette aufgedrückt. Man filtrierte bei 20-25°C über Celite und dampfte das Solvens im Vakuum ab. Der Rückstand wurde in 25 ml konz. Salzsäure gelöst und hierzu unter Rühren bei 60°C eine Lösung aus 4.3 g (35 mmol) Kaliumchlorat in 60 ml $H_2O$ zugetropft. Nach 0,5 h Nachrühren bei 60°C wurde bei 20 -25°C auf pH 6.5 mit 2N KOH eingestellt und die organischen Bestandteile viermal mit je 100 ml Essigester extrahiert. 6.0 g Produkt wurden als eine Fraktion mit dem $R_f$-

Wert von 0.91 (Dichlormethan/Kieselgel) durch Flash-Chromatographie isoliert.
Ausbeute 23 % über zwei Stufen
Fp 140°C

Beispiel 9

5-(2-Chlor-1.1.2-trifluorethoxy)-2-(4-nitrophenylazo)-pyridin

In einer gasdichten Rührapparatur wurden aus einer Gasbürette zu einer Mischung aus 244 g (1,0 mol) 5-Hydroxy-2—(4-nitrophenylazo)-pyridin, 138 g (1,0 mol) Kaliumcarbonat und 2 l trockenem DMF bei 75°C 26 l Chlortrifluorethen unter Rühren aufgedrückt. Man filtrierte über Celite und wusch mit 1 l Aceton nach. Das Lösungsmittel wurde am Rotationsverdampfer abgedampft. Der Rückstand wurde in 2.5 l Essigester aufgenommen und mit viermal je 500 ml gesättigter Kochsalzlösung gewaschen.

Nach Trocknung über Kaliumcarbonat wurde das Solvens eingedampft. Die Reinigung erfolgte durch Kurzwegdestillation im Hockvakuum.
Ausbeute 234 g (65 %) rote Kristalle
Fp 107°C

Beispiel 10

3-Amino-5,6-dichlorpyridin

77,2 g (o,4 mol) 2,3-Dichlor-5-nitropyridin wurde in 135 ml Eisessig gelöst und unter Rühren 800 ml Wasser zugesetzt. In die Mischung wurde portionsweise 111,7 g (2 Mol) Eisenpulver eingetragen - (Temperatur ≤ 50°C). Nach Beendigung der Reaktion wurde abgesaugt und das Produkt mit Ethylacetat ausgewaschen. Die organische Phase wurde mit Wasser neutral gewaschen, mit $MgSO_4$ getrocknet und eingedampft. Das Produkt wurde aus Toluol umkristallisiert.
Ausbeute 85 %
Fp 107°C

Beispiel 11

5,6-Dichlor-3-pyridinol

Verfahren a)

8,15 g (50 mmol) 3-Amino-5,6-dichlorpyridin wurden in 100 ml 8n $H_2SO_4$ gelöst und bei 0°C mit 3,55 g (53 mmol) Natriumnitrit in 9 ml Wasser diazotiert. Die kalte Diazoniumsalzlösung wurde in einer 100°C warme 60 %ige Schwefelsäure getropft. Nach Beendigung der Stickstoffabspaltung wurde neutralisiert und die Mischung mit Toluol perforiert. Die getrocknete Toluolphase wurde eingedampft und der Rückstand aus Toluol mehrfach umkristallisiert.
Fp 184 -185°C

Verfahren b)

97,8 g (0,6 Mol) 3-Amino-5,6-dichlorpyridin wurden in 900 ml 50 % $HBF_4$ gelöst und bei 0°C mit 43,5 g (o,63 Mol) Natriumnitrit in 600 ml Wasser diazotiert. Es wurde 0,5 h nachgerührt, das Diazoniumsalz abgesaugt, mit Eiswasser und Ether gewaschen. Das trockene Salz wurde dann in 600 ml Acetanhyrid langsam auf 70°C erwärmt. Nach beendeter $N_2$-Entwicklung wurde einrotiert, der Rückstand in Ether aufgenommen, mit Wasser neutralgewaschen, mit $MgSO_4$ getrocknet, eingedampft und im Vakuum destilliert. Man erhielt 3-Acetoxy-5,6-dichlorpyridin vom
Kp. 102 -103°C / 0,01 mbar

41,2 g (0,2 Mol) 3-Acetoxy-5,6-dichlorpyridin in 20 ml Methanol wurden bei Raumtemperatur zu 300 ml methanolischem KOH (2N) zugetropft und 30 Min. nachgerührt. Nach Beendigung der Reaktion wurde das Reaktionsgemsich eingedampft, der Rückstand mit 50 ml Wasser versetzt und mit Eisessig neutralisiert und getrocknet. Nach Umkristallisation aus Toluol erhielt man das Produkt mit
Ausbeute 95 %
FP 184 -185°C

Die im Verfahren b) für Verbindung 29 beschriebenen zwei getrennten Verfahrensschritte können zur Vereinfachung auch im "Eintopfverfahren" durchgeführt werden.

Beispiel 12

5,6-Dichlor-3-pyridinol-1-oxid

3,28 g (20 mmol) 5,6-Dichlor-3-pyridinol in 15 ml Eisessig wurden mit 2,14 g (21 mmol) 35 %igem Wasserstoffperoxid bei 100°C N-oxidiert. Nach beendeter Reaktion wurde neutralisiert, mit Ethylacetat extrahiert, neutral gewaschen und das Lösungsmittel abgedampft.
Ausbeute 85 %
Fp 174-175°C

Beispiel 12

3-Chlor-5-hydroxy-2-(4-nitrophenylazo)-pyridin

27,6 g (0,2 mol) 4-Nitroanilin wurden in einer Mischung aus 60 ml konz. HCl und 500 ml Wasser durch einstündiges Rühren bei 30°C gelöst. Nach Zusatz von 700 g Eis wurden bei maximal 4°C eine Lösung aus 14.6 g (0,21 mol) Natriumnitrit in 140 ml Wasser zugetropft. Man versetzte bei 0°C mit 2.6 g Aktivkohle und 5 g Kieselgel und filtrierte. Die erhaltene Lösung wurde bei pH 8 -8,5, das durch kontinuierliches Zudosieren von 2n KOH eingestellt wurde, zu einer Lösung von 25.9 g (o,2 mol) 5-Chlor-3-hydroxypyridin und 11,2 g KOH in 500 ml $H_2O$ bei 0°C unter Rühren zugetropft. Nach beendeter Zugabe rührte man 1 h bei 0°C nach und versetzte die Mischung mit 50 ml Essigsäure und erhielt so die Azoverbindung in Form roter Kristalle.
Ausbeute 52,7 g (94 %)
Fp. 207°C

Beispiel 14

5-Amino-3-chlor-2-(2,6-dichlorphenoxy)pyridin

6,39 g (20 mmol) 3-Chlor-2-(2,6-dichlorphenoxy)-5-nitropyridin wurden in 7 ml Eisessig und 40 ml Wasser suspendiert und bei Raumtemperatur 5,6 g (0,1 Mol) Eisenpulver portionsweise zugesetzt - (Temperatur 50°C). Es wurde 1 h nachgerührt, der vollständige Umsatz mit TLC bestimmt, über eine Glasfilterfritte abgesaugt und der Filterkuchen sorgfältig mit Ethylacetat gewaschen. Die organische Phase wurde mit Wasser neutral gewaschen, mit $MgSO_4$ getrocknet und eingedampft.

Beispiel 15

4-(3-Chlor-5-difluormethoxy-2-pyridyloxy)-3,5-dichloranilin

9,26 g (52 mmol) 3,5-Dichlor-4-hydroxyanilin, 10,1 g (50 mmol) 2,3-Dichlor-5-difluormethoxypyridin und 10,4 g wasserfreies Kaliumcarbonat wurden in 50 ml wasserfreiem Dimethylsulfoxid 15 h bei 105°C unter Argon gerührt. Dann wurde das Dimethylsulfoxid abdestilliert, der Rückstand in 100 ml Ether aufgenommen, zweimal mit 30 ml 5 %iger Kaliumcarbonatlösung und mit Wasser neutral gewaschen und eingedampft.
Ausbeute 14,8 g (83 %)
Rohprodukt, dkl. Öl

Beispiel 16

4-(3-Chlor-5-difluormethoxy-2-pyridyloxy)-3,5-dichlorphenyl-isocyanat

2 g (5,8 mmol) 4-(3-Chlor-5-difluormethoxy-2-pyridyloxy)-3,5-dichloranilin wurden in 25 ml wasserfreiem Toluol gelöst, bei 0°C 2,9 g (29 mmol) Phosgen eingegast und langsam auf 105°C erwärmt. Mit Inertgas wurde überschüssiges Phosgen verdrängt, das Lösungsmittel eingedampft und der Rückstand im Hochvakuum getrocknet.
Ausbeute 2,1 g (quant.); rotbraunes Öl
IR 2200 $cm^{-1}$ (N=C=O)

Gemäß den vorgenannten Herstellungsmethoden lassen sich die in nachfolgender Tabelle zusammengefaßten Verbindungen synthetisieren.

**Tabelle 1** Pyridine der Formel 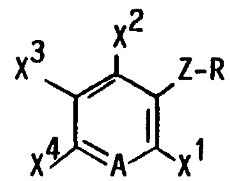 I

| Beisp. | Z | $X^1$ | $X^2$ | $X^3$ | $X^4$ | -R | A | Fp °C<br>KP °C / mbar |
|---|---|---|---|---|---|---|---|---|
| 17 | O | Cl | H | Cl | Cl | - $CF_2CHClF$ | N | |
| 18 | 0 | H | H | Cl | Cl | - $CClF_2$ | N | 95 / 0,01 |
| 19 | 0 | Cl | H | Cl | Cl | - $CClF_2$ | N | 97 / 0,01 |
| 20 | 0 | H | H | Cl | Cl | - $CF_2CHFCF_3$ | N | 100 / 0,02 |
| 21 | 0 | J | H | H | H | - $CF_2CHClF$ | N | 69 / 0,01 |
| 22 | 0 | Br | H | Cl | H | - $CF_2CHClF$ | N | 64 / 0,001 |
| 23 | 0 | Br | H | Cl | Br | - $CF_2CHClF$ | N | 97 / 0,001 |
| 24 | 0 | Br | H | H | Br | - $CF_2CHClF$ | N | 105 / 0,001 |
| 25 | 0 | H | H | Cl | H | - $CF_2CHFCF_3$ | N | 40 / 0,04 |
| 26 | 0 | H | H | H | H | - $CF_2CHFCF_3$ | N→0 | 42 - 44 |
| 27 | 0 | $NH_2$ | H | H | H | - $CF_2CHFCF_3$ | N | 40 - 41 |
| 28 | 0 | H | H | H | H | - $F_2CHClF$ | N | 84 / 10 |

**Fortsetzung Tabelle 1**

| Beisp. | Z | $X^1$ | $X^2$ | $X^3$ | $X^4$ | - R | A | Fp °C<br>Kp °C / mbar |
|---|---|---|---|---|---|---|---|---|
| 29 | O | H | H | H | Cl | $-CF_2CHFCF_3$ | N | 88 / 6 |
| 30 | O | H | H | H | OH | $-CF_2CHClF$ | N | 85 |
| 31 | O | H | H | H | $NH_2$ | $-CF_2CHF_2$ | N | 60-70/0.01 |
| 32 | O | H | H | Cl | Cl | $-CF_2CHF_2$ | N | 60-61/0,005 |
| 33 | O | H | H | H | 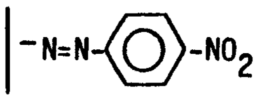 | $-CF_2CHF_2$ | N | 122-125 |
| 34 | O | H | H | H | 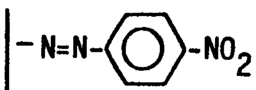 | $-CF_2CHFCF_3$ | N | 106 |
| 35 | O | H | H | H | 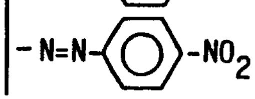 | $-CHF_2$ | N | 103-105 |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 36 | O | H | H | H | $NH_2$ | $-CF_2CHFCF_3$ | N | 74/0,1 |
| 37 | O | H | H | H | $NH_2$ | $-CHF_2$ | N | 62-65/0,1 |
| 38 | O | H | H | Br | $NH_2$ | $-CF_2CHFCl$ | N | 85/0.1 |
| 39 | O | H | H | Br | $NH_2$ | $CF_2CHFCF_3$ | N | 72-73/0.1 |
| 40 | O | H | H | Cl | $NH_2$ | $CFCHF_2$ | N | 70-72/0.1 |
| 41 | O | 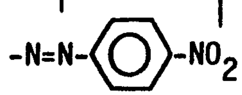 | H | Cl | H | $OCF_2H$ | N | 153-157 |
| 42 | NH | H | H | F | F | H | N | |
| 43 | NH | H | H | F | Cl | H | N | |
| 44 | NH | H | H | F | Br | H | N | |
| 45 | NH | H | H | Cl | F | H | N | |
| 46 | NH | H | H | Cl | Br | H | N | 104 |
| 47 | NH | H | H | Br | F | H | N | |
| 48 | NH | H | H | Br | Cl | H | N | 113 |
| 49 | NH | H | H | Br | Br | H | N | |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 50 | O | H | H | F | F | H | N | |
| 51 | O | H | H | F | Cl | H | N | |
| 52 | O | H | H | F | Br | H | N | |
| 53 | O | H | H | Cl | F | H | N | |
| 54 | O | H | H | Cl | Br | H | N | 185 |
| 55 | O | H | H | Br | F | H | N | |
| 56 | O | H | H | Br | Cl | H | N | 178 |
| 57 | O | H | H | Br | Br | H | N | |
| 58 | O | Br | H | Cl | Br | H | N | 180 |
| 59 | O | Br | H | F | Br | H | N | |
| 60 | O | Br | H | Br | Br | H | N | |
| 61 | O | Cl | Cl | Cl | Cl | H | N | |
| 62 | O | H | H | Br | 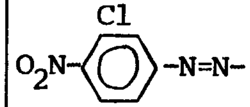 | H | N | |
| 63 | O | H | H | F | 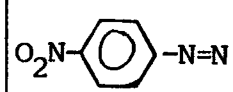 | H | N | |
| 64 | O | H | H | F | F | H | N→O | |
| 65 | O | H | H | F | Cl | H | " | |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 66 | O | H | H | F | Br | H | N→O | |
| 67 | O | H | H | Cl | F | H | " | |
| 68 | O | H | H | Cl | Br | H | " | |
| 69 | O | H | H | Br | F | H | " | |
| 70 | O | H | H | Br | Cl | H | " | |
| 71 | O | H | H | Br | Br | H | " | |
| 72 | O | H | H | F | F | $CH_3CO$ | N | |
| 73 | O | H | H | F | Cl | $CH_3CO$ | N | |
| 74 | O | H | H | F | Br | $CH_3CO$ | N | |
| 75 | O | H | H | Cl | F | $CH_3CO$ | N | |
| 76 | O | H | H | Cl | Cl | $CH_3CO$ | N | 102-3/0.01 |
| 77 | O | H | H | Cl | Br | $CH_3CO$ | N | 103-8/0.01 |
| 78 | O | H | H | Br | F | $CH_3CO$ | N | |
| 79 | O | H | H | Br | Cl | $CH_3CO$ | N | 103 /0.01 |
| 80 | O | H | H | Br | Br | $CH_3CO$ | N | |
| 81 | O | H | H | F | F | $CH_3CO$ | N→O | |
| 82 | O | H | H | F | Cl | $CH_3CO$ | " | |
| 83 | O | H | H | F | Br | $CH_3CO$ | " | |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 84 | O | H | H | Cl | F | $CH_3CO$ | N→O | |
| 85 | O | H | H | Cl | Cl | $CH_3CO$ | " | |
| 86 | O | H | H | Cl | Br | $CH_3CO$ | " | |
| 87 | O | H | H | Br | F | $CH_3CO$ | " | |
| 88 | O | H | H | Br | Cl | $CH_3CO$ | " | |
| 89 | O | H | H | Br | Br | $CH_3CO$ | " | |
| 90 | O | H | H | H | H | $CHF_2$ | N | 114/80 |
| 91 | O | H | H | H | H | $CF_2Cl$ | N | |
| 92 | O | H | H | H | H | $CF_3$ | N | |
| 93 | O | H | H | H | H | $CF_2CHF_2$ | N | |
| 94 | O | H | H | H | H | $CF_2CHFBr$ | N | |
| 95 | O | H | H | H | H | $CH_2CF_3$ | N | |
| 96 | O | H | H | H | H | $CF_2CHFCF_3$ | N | |
| 97 | O | H | H | H | H | $CHF_2$ | N→O | |
| 98 | O | H | H | H | H | $CF_2Cl$ | " | |
| 99 | O | H | H | H | H | $CF_3$ | " | |
| 100 | O | H | H | H | H | $CF_2CHF_2$ | " | |
| 101 | O | H | H | H | H | $CF_2CHFCl$ | " | |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 102 | O | H | H | H | H | $CF_2CHFBr$ | N→O | |
| 103 | O | H | H | H | F | $CF_2H$ | N | 60 / 18 |
| 104 | O | H | H | H | F | $CF_2Cl$ | N | |
| 105 | O | H | H | H | F | $CF_3$ | N | |
| 106 | O | H | H | H | F | $CF_2CHF_2$ | N | |
| 107 | O | H | H | H | F | $CF_2CHFCl$ | N | Öl |
| 108 | O | H | H | H | F | $CF_2CHFBr$ | N | |
| 109 | O | H | H | H | F | $CH_2CF_3$ | N | |
| 110 | O | H | H | H | F | $CF_2CHFCF_3$ | N | |
| 111 | O | H | H | H | Cl | $CHF_2$ | N | |
| 112 | O | H | H | H | Cl | $CF_2Cl$ | N | |
| 113 | O | H | H | H | Cl | $CF_3$ | N | |
| 114 | O | H | H | H | Cl | $CH_2CF_3$ | N | |
| 115 | O | H | H | H | Br | $CF_2H$ | N | |
| 116 | O | H | H | H | Br | $CF_2Cl$ | N | |
| 117 | O | H | H | H | Br | $CF_3$ | N | |
| 118 | O | H | H | H | Br | $CF_2CHF_2$ | N | |
| 119 | O | H | H | H | Br | $CF_2CHFCl$ | N | |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 120 | O | H | H | H | Br | $CF_2CHFBr$ | N | |
| 121 | O | H | H | H | Br | $CH_2CF_3$ | N | |
| 122 | O | H | H | H | Br | $CF_2CHFCF_3$ | N | |
| 123 | O | H | H | H | Cl | $CF_2H$ | N→O | |
| 124 | O | H | H | H | Cl | $CF_2Cl$ | " | |
| 125 | O | H | H | H | Cl | $CF_3$ | " | |
| 126 | O | H | H | H | F | $CF_2CHF_2$ | " | |
| 127 | O | H | H | H | F | $CF_2CHFCl$ | " | |
| 128 | O | H | H | H | Cl | $CF_2CHFBr$ | " | |
| 129 | O | H | H | H | Cl | $CH_2CF_3$ | " | |
| 130 | O | H | H | H | Br | $CF_2CHFCF_3$ | " | |
| 131 | O | Br | H | H | H | $CF_2CHFCl$ | N | 85-95/0.08 |
| 132 | O | Br | H | Cl | H | $CHF_2$ | N | 46-8 /0.01 |
| 133 | O | H | H | Cl | H | $CHF_2$ | N | 32 / 0.01 |
| 134 | O | H | H | F | F | $CHF_2$ | N | |
| 135 | O | H | H | F | F | $CF_2Cl$ | N | |
| 136 | O | H | H | F | F | $CF_3$ | N | |
| 137 | O | H | H | F | F | $CF_2CHF_2$ | N | |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 138 | O | H | H | F | F | $CF_2CHFCl$ | N | |
| 139 | O | H | H | F | F | $CF_2CHFBr$ | N | |
| 140 | O | H | H | F | F | $CH_2CF_3$ | N | |
| 141 | O | H | H | F | F | $CF_2CHFCF_3$ | N | |
| 142 | O | H | H | F | Cl | $CHF_2$ | N | |
| 143 | O | H | H | F | Cl | $CF_2Cl$ | N | |
| 144 | O | H | H | F | Cl | $CF_3$ | N | |
| 145 | O | H | H | F | Cl | $CF_2CHF_2$ | N | |
| 146 | O | H | H | F | Cl | $CF_2CHClF$ | N | |
| 147 | O | H | H | F | Cl | $CF_2CHFBr$ | N | |
| 148 | O | H | H | F | Cl | $CH_2CF_3$ | N | |
| 149 | O | H | H | F | Cl | $CF_2CHFCF_3$ | N | |
| 150 | O | H | H | F | Br | $CHF_2$ | N | |
| 151 | O | H | H | F | Br | $CF_2Cl$ | N | |
| 152 | O | H | H | F | Br | $CF_3$ | N | |
| 153 | O | H | H | F | Br | $CF_2CHF_2$ | N | |
| 154 | O | H | H | F | Br | $CF_2CHFCl$ | N | |
| 155 | O | H | H | F | Br | $CF_2CHFBr$ | N | |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 156 | O | H | H | F | Br | $CH_2CF_3$ | N | |
| 157 | O | H | H | F | Br | $CF_2CHFCF_3$ | N | |
| 158 | O | H | H | Cl | F | $CHF_2$ | N | |
| 159 | O | H | H | Cl | F | $CF_2Cl$ | N | |
| 160 | O | H | H | Cl | F | $CF_3$ | N | |
| 161 | O | H | H | Cl | F | $CF_2CHF_2$ | N | |
| 162 | O | H | H | Cl | F | $CF_2CHFCl$ | N | |
| 163 | O | H | H | Cl | F | $CF_2CHFBr$ | N | |
| 164 | O | H | H | Cl | F | $CH_2CF_3$ | N | |
| 165 | O | H | H | Cl | F | $CF_2CHFCF_3$ | N | |
| 166 | O | H | H | Cl | Cl | $CF_3$ | N | 66-7/15 |
| 167 | O | H | H | Cl | Cl | $CF_2CHFBr$ | N | |
| 168 | O | H | H | Cl | Cl | $CH_2CF_3$ | N | |
| 169 | O | H | H | Cl | Br | $CHF_2$ | N | 68-70/0.1 |
| 170 | O | H | H | Cl | Br | $CF_2Cl$ | N | |
| 171 | O | H | H | Cl | Br | $CF_3$ | N | |
| 172 | O | H | H | Cl | Br | $CF_2CHF_2$ | N | |
| 173 | O | H | H | Cl | Br | $CF_2CHFCl$ | N | |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 174 | O | H | H | Cl | Br | $CF_2CHFBr$ | N | |
| 175 | O | H | H | Cl | Br | $CH_2CF_3$ | N | |
| 176 | O | H | H | Cl | Br | $CF_2CHFCF_3$ | N | |
| 177 | O | H | H | Br | F | $CHF_2$ | N | |
| 178 | O | H | H | Br | F | $CF_2Cl$ | N | |
| 179 | O | H | H | Br | F | $CF_3$ | N | |
| 180 | O | H | H | Br | F | $CF_2CHF_2$ | N | |
| 181 | O | H | H | Br | F | $CF_2CHFCl$ | N | |
| 182 | O | H | H | Br | F | $CF_2CHFBr$ | N | |
| 183 | O | H | H | Br | F | $CH_2CF_3$ | N | |
| 184 | O | H | H | Br | F | $CF_2CHFCF_3$ | N | |
| 185 | O | H | H | Br | Cl | $CF_2H$ | N | 100/0.01 |
| 186 | O | H | H | Br | Cl | $CF_2Cl$ | N | |
| 187 | O | H | H | Br | Cl | $CF_3$ | N | |
| 188 | O | H | H | Br | Cl | $CF_2CHF_2$ | N | |
| 189 | O | H | H | Br | Cl | $CF_2CHFCl$ | N | 123/24 |
| 190 | O | H | H | Br | Cl | $CF_2CHFBr$ | N | |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 191 | O | H | H | Br | Cl | $CH_2CF_3$ | N | |
| 192 | O | H | H | Br | Cl | $CF_2CHFCF_3$ | N | |
| 193 | O | H | H | Br | Br | $CHF_2$ | N | |
| 194 | O | H | H | Br | Br | $CF_2Cl$ | N | |
| 195 | O | H | H | Br | Br | $CF_3$ | N | |
| 196 | O | H | H | Br | Br | $CF_2CHF_2$ | N | |
| 197 | O | H | H | Br | Br | $CF_2CHFCl$ | N | |
| 198 | O | H | H | Br | Br | $CF_2CHFBr$ | N | |
| 199 | O | H | H | Br | Br | $CH_2CF_3$ | N | |
| 200 | O | H | H | Br | Br | $CF_2CHFCF_3$ | N | |
| 201 | O | H | H | Cl | Cl | $CH_2C{\equiv}CCl$ | N | |
| 202 | O | H | H | Br | F | $CH_2{-}CCl{=}CHCl$ | N | |
| 203 | O | H | H | Cl | Br | $CH_2{-}CH{=}CHCl$ | N | |
| 204 | O | Br | H | H | Br | $CF_2CHFCF_3$ | N | 135-7/18 |
| 205 | O | H | H | F | F | $CHF_2$ | N→O | |
| 206 | O | H | H | Cl | F | $CF_2Cl$ | N→O | |
| 207 | O | H | H | Br | F | $CF_3$ | N→O | |
| 208 | O | H | H | F | Cl | $CF_2CHF_2$ | N→O | |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 209 | O | H | H | Cl | Cl | $CF_2CHFCl$ | N→O | 80-3/0.0001 |
| 210 | O | H | H | Br | Cl | $CF_2CHFBr$ | N→O | |
| 211 | O | H | H | Cl | H | $CF_2CHFCl$ | N→O | 100-101/0.01 |
| 212 | O | H | H | Br | Br | $CF_2CHFCF_3$ | N→O | |
| 213 | O | H | H | H | $NH_2$ | $CF_2Cl$ | N | |
| 214 | O | H | H | H | $NH_2$ | $CF_3$ | N | |
| 215 | O | H | H | H | $NH_2$ | $CF_2CHBrF$ | N | |
| 216 | O | H | H | H | $NH_2$ | $CH_2CF_3$ | N | |
| 217 | O | H | H | Cl | $NH_2$ | $CF_2H$ | N | 60/0.05 |
| 218 | O | H | H | Cl | $NH_2$ | $CF_2Cl$ | N | |
| 219 | O | H | H | Cl | $NH_2$ | $CF_3$ | N | |
| 220 | O | H | H | Cl | $NH_2$ | $CF_2CHFCl$ | N | |
| 221 | O | H | H | Cl | $NH_2$ | $CF_2CHFBr$ | N | |
| 222 | O | H | H | Cl | $NH_2$ | $CH_2CF_3$ | N | |
| 223 | O | H | H | Cl | $NH_2$ | $CF_2CHFCF_3$ | N | |
| 224 | O | H | H | Br | $NH_2$ | $CHF_2$ | N | |
| 225 | O | H | H | Br | $NH_2$ | $CF_2Cl$ | N | |
| 226 | O | H | H | Br | $NH_2$ | $CF_3$ | N | |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 227 | O | H | H | Br | $NH_2$ | $CF_2CHF_2$ | N | |
| 228 | O | H | H | Br | $NH_2$ | $CF_2CHFBr$ | N | |
| 229 | O | H | H | Br | $NH_2$ | $CH_2CF_3$ | N | |
| 230 | O | H | H | F | $NH_2$ | $CF_3$ | N | |
| 231 | O | $NH_2$ | H | H | H | $CHF_2$ | N | 50/0.3 |
| 232 | O | H | H | H | OH | $CHF_2$ | N | 99-102 |
| 233 | O | H | H | H | OH | $CF_2Cl$ | N | |
| 234 | O | H | H | H | OH | $CF_3$ | N | |
| 235 | O | H | H | H | OH | $CF_2CHFBr$ | N | |
| 236 | O | H | H | H | OH | $CH_2CF_3$ | N | |
| 237 | O | H | H | H | OH | $CF_2CHFCF_3$ | N | 79-84 |
| 238 | O | H | H | Cl | OH | $CHF_2$ | N | 140-143 |
| 239 | O | H | H | Cl | OH | $CF_2Cl$ | N | |
| 240 | O | H | H | Cl | OH | $CF_3$ | N | |
| 241 | O | H | H | Cl | OH | $CF_2CHF_2$ | N | 150-3 |
| 242 | O | H | H | Cl | OH | $CF_2CHFBr$ | N | |
| 243 | O | H | H | Cl | OH | $CH_2CF_3$ | N | |
| 244 | O | H | H | Cl | OH | $CF_2CHFCF_3$ | N | 113-4 |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 245 | O | H | H | Br | OH | $CHF_2$ | N | |
| 246 | O | H | H | Br | OH | $CF_2Cl$ | N | |
| 247 | O | H | H | Br | OH | $CF_3$ | N | |
| 248 | O | H | H | Br | OH | $CF_2CHF_2$ | N | |
| 249 | O | H | H | Br | OH | $CF_2CHFCl$ | N | Öl |
| 250 | O | H | H | Br | OH | $CF_2CHFBr$ | N | |
| 251 | O | H | H | Br | OH | $CH_2CF_3$ | N | |
| 252 | O | H | H | Br | OH | $CF_2CHFCF_3$ | N | |
| 253 | O | H | H | H | 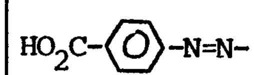 | $CF_2CHFCl$ | N | Öl |
| 254 | O | H | H | Cl | 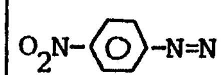 | $CHF_2$ | N | Öl |
| 255 | O | H | H | H | 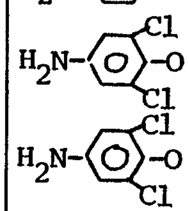 | $CHF_2$ | N | |
| 256 | O | H | H | H | | $CF_2Cl$ | N | |
| 257 | O | H | H | H | " | $CF_3$ | N | |
| 258 | O | H | H | H | " | $CF_2CHF_2$ | N | |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 259 | O | H | H | H | 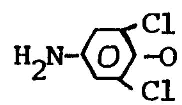 | $CF_2CHFCl$ | N | Öl ($n_D^{20}$ 1.5865) |
| 260 | O | H | H | H | " | $CF_2CHFBr$ | N | |
| 261 | O | H | H | H | " | $CH_2CF_3$ | N | |
| 262 | O | H | H | H | " | $CF_2CHFCF_3$ | N | |
| 263 | O | H | H | F | " | $CHF_2$ | N | |
| 264 | O | H | H | Cl | " | $CF_2Cl$ | N | 98-99 |
| 265 | O | H | H | Cl | " | $CF_3$ | N | 83-5 |
| 266 | O | H | H | Cl | " | $CF_2CHF_2$ | N | Öl ($n_D^{20}$ 1.5415) |
| 267 | O | H | H | Cl | " | $CF_2CHFCl$ | N | Öl ($n_D^{20}$ 1.5963) |
| 268 | O | H | H | F | " | $CF_2CHFBr$ | N | |
| 269 | O | H | H | Br | " | $CH_2CF_3$ | N | |
| 270 | O | H | H | Cl | " | $CF_2CHFCF_3$ | N | Öl ($n_D^{20}$ 1.5873) |
| 271 | O | H | H | Br | " | $CF_2CHFCl$ | N | Öl ( " 1.5921) |
| 272 | O | H | H | Br | " | $CHF_2$ | N | Öl ( " 1.5798) |
| 273 | O | 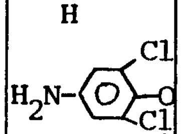 | H | Cl | H | $CHF_2$ | N | Öl ( " 1.5908) |
| 274 | O | 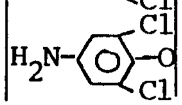 | H | Cl | H | $CF_2CHFCl$ | N | Öl ( " 1.5763) |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 275 | O | H | H | F | 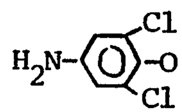 | $CHF_2$ | N→O | |
| 276 | O | H | H | Cl | " | $CF_2Cl$ | N→O | |
| 277 | O | H | H | Br | " | $CF_3$ | N→O | |
| 278 | O | H | H | F | " | $CF_2CHF_2$ | N→O | |
| 279 | O | H | H | Cl | " | $CF_2CHCl$ | N→O | |
| 280 | O | H | H | Br | " | $CF_2CHFBr$ | N→O | |
| 281 | O | H | H | F | " | $CH_2CF_3$ | N→O | |
| 282 | O | H | H | Cl | " | $CF_2CHFCF_3$ | N→O | |
| 283 | O | H | H | Cl | 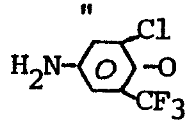 | $CHF_2$ | N | |
| 284 | O | H | H | Br | " | $CF_3$ | N→O | |
| 285 | O | H | H | F | " | $CF_2CHF_2$ | N | |
| 286 | O | H | H | Cl | " | $CF_2CHFCF_3$ | N | |
| 287 | O | H | H | F | 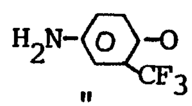 | $CF_2H$ | N | |
| 288 | O | H | H | Cl | " | $CF_3$ | N→O | |
| 289 | O | H | H | Br | " | $CF_2CHF_2$ | N | |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 290 | O | H | H | Cl | 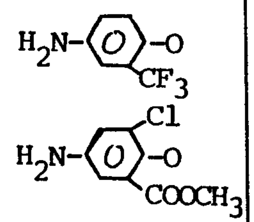 | $CF_2CHFCF_3$ | N→O | |
| 291 | O | H | H | F | | $CF_2H$ | N | |
| 292 | O | H | H | Cl | " | $CF_3$ | N→O | |
| 293 | O | H | H | Br | " | $CF_2CHF_2$ | N | |
| 294 | O | H | H | Cl | " | $CF_2CHFCF_3$ | N→O | |
| 295 | O | H | H | F | 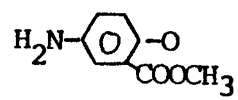 | $CF_2H$ | N | |
| 296 | O | H | H | Cl | " | $CF_3$ | N→O | |
| 297 | O | H | H | Br | " | $CF_2CHF_2$ | N | |
| 298 | O | H | H | Cl | " | $CF_2CHFCF_3$ | N→O | |
| 299 | O | H | H | Cl | 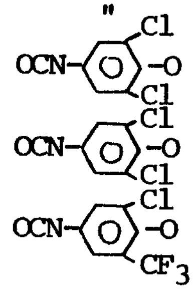 | $CF_3$ | N | |
| 300 | O | H | H | Cl | | $CF_2H$ | N→O | |
| 301 | O | H | H | H | | $CF_2CHF_2$ | N | |

**Fortsetzung Tabelle 1**

| Beisp. | Z | $X^1$ | $X^2$ | $X^3$ | $X^4$ | R | A | Fp °C<br>Kp °C / mbar |
|---|---|---|---|---|---|---|---|---|
| 302 | O | H | H | H | 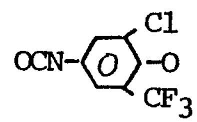 | $CF_2CHFCl$ | N→O | |
| 303 | O | H | H | H | 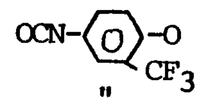 | $CF_2CHFCF_3$ | N | |
| 304 | O | H | H | H | " | $CF_3$ | N→O | |
| 305 | O | H | H | H | 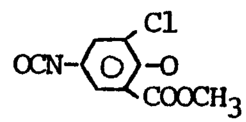 | $CF_2H$ | N | |
| 306 | O | H | H | H | " | $CF_2CHFCF_3$ | N→O | |
| 307 | O | H | H | H | 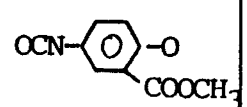 | $CF_2CHFCF_3$ | N | |
| 308 | O | H | H | H | " | $CHF_2$ | N→O | |

**Ansprüche**

1. Verbindungen der Formel I

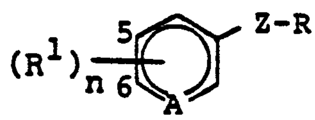

(I),

worin
A = N oder N→O
Z = O oder NH,
R = H, halogeniertes ($C_1$-$C_8$)Alkyl, halogeniertes ($C_2$-$C_8$)Alkenyl oder halogeniertes ($C_2$-$C_6$)Alkinyl, sowie für Z = O ($C_1$-$C_4$)Alkoxycarbonyl
$R^1$ = bei gleicher oder verschiedener Bedeutung Wasserstoff, Halogen, Amino, -NHOH Hydroxy, Phenylazo, das im Phenylring ein-oder mehrfach durch Halogen, Nitro, Carboxy, Sulfo,($C_1$-$C_8$)-Alkyl, ($C_1$-$C_8$)-Alkoxy, halogeniertes ($C_1$-$C_8$)Alkyl oder halogeniertes ($C_1$-$C_8$)Alkoxy substituiert sein kann,
einen Rest der Formeln
Y = C = N-, $Y^1Y^2$C = N-, $Y^3$NH-oder

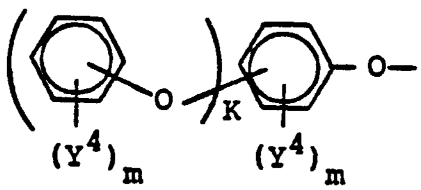

Y = O oder S
$Y^1$ = Halogen, ($C_1$-$C_3$)Alkylthio, ($C_1$-$C_3$)Alkylsulfenyl, ($C_1$-$C_3$)Alkylsulfonyl,
$Y^2$ = einen für $Y^1$ genannten Rest oder Mercapto,
$Y^3$ = ($C_1$-$C_6$)Alkyl, ($C_1$-$C_6$)Alkylsulfenyl, die beide halogeniert sein können, Phenylsulfonyl, das durch ($C_1$-$C_6$)-Alkyl, ($C_1$-$C_6$)Alkoy, ($C_1$-$C_6$)Alkoxy-carbonyl, Halo($C_1$-$C_6$)alkyl, Halo($C_1$-$C_6$)alkoxy, Halo($C_1$-$C_6$-alkyl)carbonyl, Halogen, Nitro oder Cyano substi tuiert sein kann, Phosphoryl oder Thiophosphoryl, die beide durch zwei Reste der Gruppe ($C_1$-$C_8$)Alkoxy, ($C_1$-$C_6$)Alkylthio, Amino, ($C_1$-$C_6$)Alkylamino oder Di($C_1$-$C_6$alkyl)amino substituiert sein können,
$Y^4$ = unabhängig voneinander H, Halogen, $NO_2$, $NH_2$, -$NHY^3$, -NHOH, -NCO, NCS, OH, ($C_1$-$C_6$)Alkyl, ($C_2$-$C_6$)-Alkenyl, ($C_2$-$C_6$)Alkinyl, ($C_1$-$C_6$)Alkoxy, ($C_1$-$C_6$)Alkoxy-carbonyl, wobei die fünf letzt genannten Reste halogeniert sein können, Nitro, Cyano, Carboxy oder ein Carboxylat-Salz,
K = 0 oder 1,
m,n = unabhängig voneinander eine Zahl von 1 bis 4 bedeuten, mit der Maßgabe, daß, wenn Z-R = OH oder $NH_2$ bedeutet,n≠o ist und mindestens ein Rest $R^1$ = einen Rest der Formel

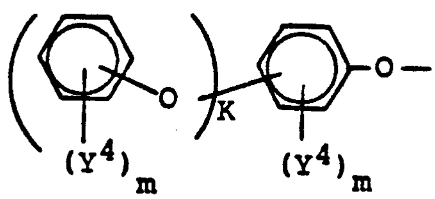

bedeutet oder $(R^1)_n$ für zwei Rests in 5-oder 6-Position des heterocyclischen Ringes steht, und zwar in Position 5 für Halogen und in Position 6 für einen Rest der Gruppe Halogen, Amino, Hydroxy oder Phenylazo, das wie oben angegeben substituiert sein kann.

2. Verbindungen der Formel I, worin
A = N oder N→O
Z = O oder NH,
R = H, halogeniertes ($C_1$-$C_8$)Alkyl,
$R^1$ bei gleicher oder verschiedener Bedeutung Wasserstoff, Halogen, Amino, Hydroxy, Phenylazo, das im

Phenylring ein-oder mehrfach durch Halogen, Nitro oder Carboxy substituiert sein kann, einen Rest der Formeln
Y = C = N-, $Y^1Y^2C$ = N-oder

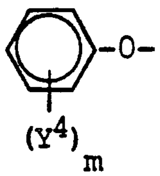

,

Y = O oder S

$Y^1$ = Halogen oder $(C_1-C_3)$Alkylthio,

$Y^2$ = einen für $Y^1$ genannten Rest oder Mercapto,

$Y^4$ = unabhängig voneinander H, $NO_2$, $NH_2$, -NHOH, Halogen, -NCO, NCS, OH, $(C_1-C_6)$Alkyl, $(C_1-C_6)$Alkoxycarbonyl, wobei die fünf letzt genannten Reste halogeniert sein können, und

m,n = unabhängig voneinander eine Zahl von 1 bis 4 bedeuten.

3. Verbindungen der Formel I, worin

A = N

Z = O oder NH,

R = H oder halogeniertes $(C_1-C_4)$Alkyl

$R^1$ = H, Halogen, Amino, Hydroxy oder

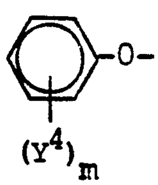

$Y^4$ = H, Cl, $CF_3$, $NH_2$, -NCO oder $(C_1-C_2)$Alkoxycarbonyl, die in Position 2, 4 oder 6 des Phenoxyrestes orientiert sind, und

m,n = eine Zahl von 1 bis 3 bedeuten.

bedeuten.

4. Verfahren zur Herstellung von Verbindungen der Formel I von Ansprüchen 1, 2 oder 3 dadurch gekennzeichnet, daß man

a) zur Herstellung von Verbindungen mit Z = O und R≠H eine Verbindung der Formel (II)

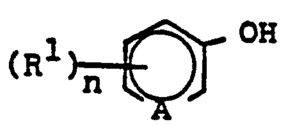

(II)

$a_1$) mit einer Verbindung der Formel $Y^1$-R', worin R' die Bedeutung von R außer H und $Y^1$ einen nucleofugen Rest wie Halogen, Phenylsulfonyl, Phenylsulfinyl, Tosyl oder Mesyl bedeutet, in Gegenwart einer Base oder

$a_2$) mit einem halogenierten $(C_2-C_8)$Alken oder halogeniertem $(C_2-C_8)$Alkin in Gegenwart einer Base, oder

$a_3$) einem halogeniertem Carben der Formel $C(Hal)_2$), wobei Hal bei gleicher oder verschiedener Bedeutung Halogen, insbesondere F oder Cl bedeuet, oder

$a_4$) mit einer Verbindung der Formel $Y^1$-CO-$R^2$, worin $R^2$ = Halogen, halogeniertes $(C_1-C_7)$Alkyl, halogeniertes $(C_2-C_7)$Alkenyl oder halogeniertes $(C_2-C_7)$Alkinyl bedeutet, umsetzt und das erhaltene Zwischenprodukt der Formel (III)

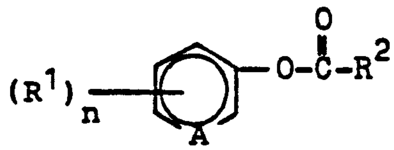

(III)

halogeniert, oder

$a_5$) zur Herstellung von Verbindungen mit Z=O und R= perhalogeniertes ($C_1$-$C_8$)Alkyl eine Verbindung der Formel (II) mit einem perhalogenierten ($C_1$-$C_8$)Alkan in Gegenwart einer Base oder in Gegenwart von HF, oder

b) zur Herstellung von Verbindungen der Formel I mit Z=O und R≠H eine Verbindung der Formel -(IV)

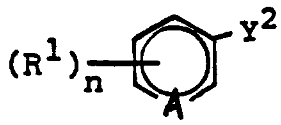

(IV),

worin $Y^2$ eine Abgangsgruppe wie Halogen, Nitro, Diazonium, Alkylsulfonyl, halogeniertes Alkylsulfonyl, Phenylsulfonyl, das im Phenylrest beispielsweise durch Alkyl, Halogen substituiert sein kann, bedeutet, mit einem Alkalisalz einer Verbindung der Formel $OH-CH_2-R^3$, worin $R^3$ die Bedeutung von $R^2$ außer Halogen besitzt, umsetzt oder

c) zur Herstellung von Verbindungen der Formel I mit Z=O und R=H eine Verbindung der Formel -(V),

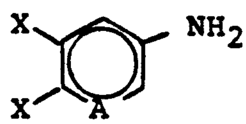

wobei X= unabhängig voneinander Halogen bedeutet, diazotiert und hydrolysiert oder

d) zur Herstellung von Verbindungen mit Z-R = $NH_2$ eine Verbindung der Formel (VI)

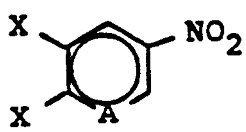

(VI)

reduziert, oder

e) zur Herstellung von Verbindungen der Formel I, die in Position 6 einen, gegebenenfalls wie oben angegeben substituierten Phenylazo-Rest enthalten, eine Verbindung der Formel (VII)

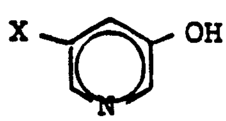

(VII)

mit einem Phenyldiazonium-Ion, das, wie für Phenylazo von Formel (I) angegeben, substituiert sein kann, umsetzt, oder

f) zur Herstellung von Verbindungen mit

$R^1$=

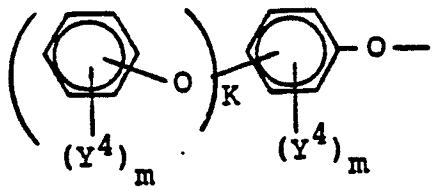

eine Verbindung der Formel (VIII)

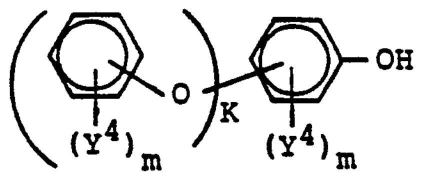

(VIII)

mit einer Verbindung der Formel IX

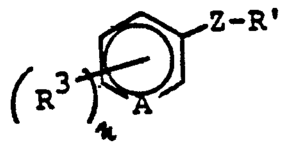

worin $R^3$ eine nucleofuge Abgangsgruppe wie Halogen, Alkylsulfinyl, Phenylsulfinyl, Phenylsulfonyl oder Alkylsulfonyl bedeutet, und R' die Bedeutung von R außer Wasserstoff besitzt, umsetzt,
und die unter a) bis f) erhaltenen Produkte gegebenenfalls derivatisiert.

5. Mittel, enthaltend eine Verbindung der Formel I von Ansprüchen 1, 2 oder 3.

6. Verwendung von Verbindungen der Formel I von Ansprüchen 1, 2 oder 3 zur Herstellung von Pflanzenschutzmitteln.

Patentansprüche für folgenden Vertragsstaat: Österreich:

1. Verfahren zur Herstellung der Verbindungen der Formel I

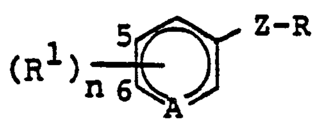

(I),

worin
A = N oder N→O
Z = O oder NH,
R = H, halogeniertes $(C_1$-$C_8)$Alkyl, halogeniertes $(C_2$-$C_8)$Alkenyl oder halogeniertes $(C_2$-$C_6)$Alkinyl, sowie für Z = O $(C_1$-$C_4)$Alkoxycarbonyl
$R^1$ = bei gleicher oder verschiedener Bedeutung Wasserstoff, Halogen, Amino, -NHOH Hydroxy, Phenylazo, das im Phenylring ein-oder mehrfach durch Halogen, Nitro, Carboxy, Sulfo,$(C_1$-$C_8)$-Alkyl, $(C_1$-$C_8)$-Alkoxy, halogeniertes $(C_1$-$C_8)$Alkyl oder halogeniertes $(C_1$-$C_8)$Alkoxy substituiert sein kann,
einen Rest der Formeln
Y = C = N-, $Y^1Y^2$C = N-, $Y^3$NH-oder

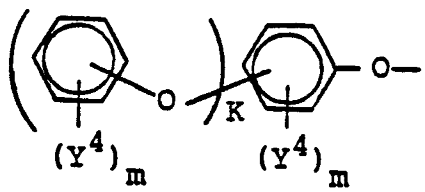

Y = O oder S

$Y^1$ = Halogen, $(C_1\text{-}C_3)$Alkylthio, $(C_1\text{-}C_3)$Alkylsulfenyl, $(C_1\text{-}C_3)$Alkylsulfonyl,

$Y^2$ = einen für $Y^1$ genannten Rest oder Mercapto,

$Y^3$ = $(C_1\text{-}C_6)$Alkyl, $(C_1\text{-}C_6)$Alkylsulfenyl, die beide halogeniert sein können, Phenylsulfenyl, das durch $(C_1\text{-}C_6)$-Alkyl, $(C_1\text{-}C_6)$Alkoy, $(C_1\text{-}C_6)$Alkoxy-carbonyl, Halo$(C_1\text{-}C_6)$alkyl, Halo$(C_1\text{-}C_6)$alkoxy, Halo$(C_1\text{-}C_6$alkyl)carbonyl, Halogen, Nitro oder Cyano substi tuiert sein kann, Phosphoryl oder Thiophosphoryl, die beide durch zwei Reste der Gruppe $(C_1\text{-}C_8)$Alkoxy, $(C_1\text{-}C_6)$Alkylthio, Amino, $(C_1\text{-}C_6)$Alkylamino oder Di$(C_1\text{-}C_6$alkyl)amino substituiert sein können,

$Y^4$ = unabhängig voneinander H, Halogen, $NO_2$, $NH_2$, $-NHY^3$, -NHOH, -NCO, NCS, OH, $(C_1\text{-}C_6)$Alkyl, $(C_2\text{-}C_6)$-Alkenyl, $(C_2\text{-}C_6)$Alkinyl, $(C_1\text{-}C_6)$Alkoxy, $(C_1\text{-}C_6)$Alkoxy-carbonyl, wobei die fünf letzt genannten Reste halogeniert sein können, Nitro, Cyano, Carboxy oder ein Carboxylat-Salz,

K = 0 oder 1,

m,n = unabhängig voneinander eine Zahl von 1 bis 4 bedeuten,

dadurch gekennzeichnet, daß man

a) zur Herstellung von Verbindungen mit Z = O und R≠H eine Verbindung der Formel (II)

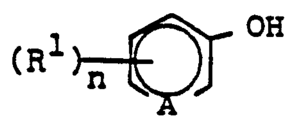

(II)

$a_1$) mit einer Verbindung der Formel $Y^1$-R', worin R' die Bedeutung von R außer H und $Y^1$ einen nucleofugen Rest wie Halogen, Phenylsulfonyl, Phenylsulfinyl, Tosyl oder Mesyl bedeutet, in Gegenwart einer Base oder

$a_2$) mit einem halogenierten $(C_2\text{-}C_8)$Alken oder halogeniertem $(C_2\text{-}C_8)$Alkin in Gegenwart einer Base, oder

$a_3$)einem halogeniertem Carben der Formel $C(Hal)_2$), wobei Hal bei gleicher oder verschiedener Bedeutung Halogen, insbesondere F oder Cl bedeutet, oder

$a_4$) mit einer Verbindung der Formel $Y^1$-CO-$R^2$, worin $R^2$ = Halogen, halogeniertes $(C_1\text{-}C_7)$Alkyl, halogeniertes $(C_2\text{-}C_7)$Alkenyl oder halogeniertes $(C_2\text{-}C_7)$Alkinyl bedeutet, umsetzt und das erhaltene Zwischenprodukt der Formel (III)

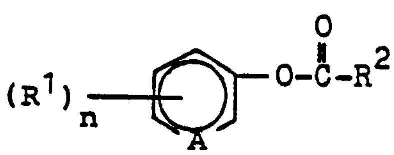

(III)

halogeniert, oder

$a_5$) zur Herstellung von Verbindungen mit Z = O und R = perhalogeniertes $(C_1\text{-}C_8)$Alkyl eine Verbindung der Formel (II) mit einem perhalogenierten $(C_1\text{-}C_8)$Alkan in Gegenwart einer Base oder in Gegenwart von HF, oder

b) zur Herstellung von Verbindungen der Formel I mit Z = O und R≠H eine Verbindung der Formel - (IV)

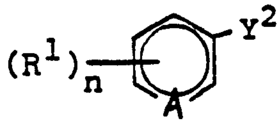

(IV),

worin $Y^2$ eine Abgangsgruppe wie Halogen, Nitro, Diazonium, Alkylsulfonyl, halogeniertes Alkylsulfonyl, Phenylsulfonyl, das im Phenylrest beispielsweise durch Alkyl, Halogen substituiert sein kann, bedeutet, mit einem Alkalisalz einer Verbindung der Formel $OH-CH_2-R^3$, worin $R^3$ die Bedeutung von $R^2$ außer Halogen besitzt, umsetzt oder

c) zur Herstellung von Verbindungen der Formel I mit Z = O und R = H eine Verbindungen der Formel (V),

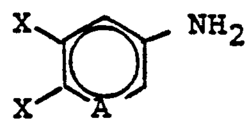

wobei X = unabhängig voneinander Halogen bedeutet, diazotiert und hydrolysiert oder

d) zur Herstellung von Verbindungen mit Z-R = $NH_2$ eine Verbindung der Formel (VI)

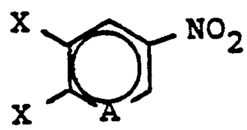

(VI)

reduziert, oder

e) zur Herstellung von Verbindungen der Formel I, die in Position 6 einen, gegebenenfalls wie oben angegeben substituierten Phenylazo-Rest enthalten, eine Verbindung der Formel (VII)

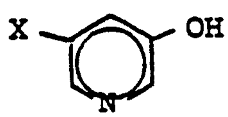

(VII)

mit einem Phenyldiazonium-Ion, das, wie für Phenylazo von Formel (I) angegeben, substituiert sein kann, umsetzt, oder

f) zur Herstellung von Verbindungen mit

$R^1 =$

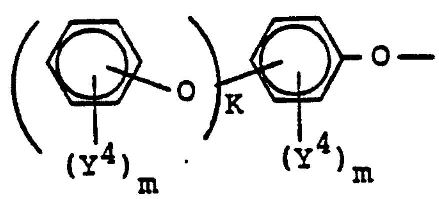

eine Verbindung der Formel (VIII)

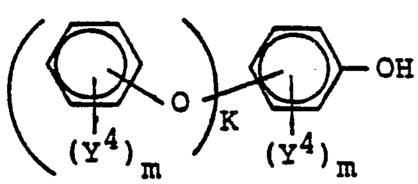

(VIII)

mit einer Verbindung der Formel IX

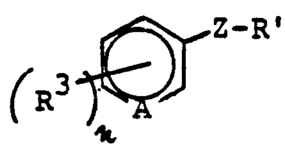

worin $R^3$ eine nucleofuge Abgangsgruppe wie Halogen, Alkylsulfinyl, Phenylsulfinyl, Phenylsulfonyl oder Alkylsulfonyl bedeutet, und R' die Bedeutung von R außer Wasserstoff besitzt, umsetzt, und die unter a) bis f) erhaltenen Produkte gegebenenfalls derivatisiert.

2. Verfahren gemäß Anspruch 1, wobei in Formel I
A = N oder N→O
Z = O oder NH,
R = H, halogeniertes $(C_1\text{-}C_8)$Alkyl,
$R^1$ bei gleicher oder verschiedener Bedeutung Wasserstoff, Halogen, Amino, Hydroxy, Phenylazo, das im Phenylring ein-oder mehrfach durch Halogen, Nitro oder Carboxy substituiert sein kann,
einen Rest der Formeln
Y = C = N-, $Y^1Y^2C$ = N-oder

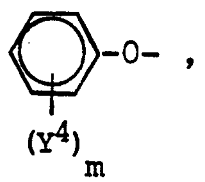

Y = O oder S
$Y^1$ = Halogen oder $(C_1\text{-}C_3)$Alkylthio,
$Y^2$ = einen für $Y^1$ genannten Rest oder Mercapto,
$Y^4$ = unabhängig voneinander H, $NO_2$, $NH_2$, -NHOH, Halogen, -NCO, NCS, OH, $(C_1\text{-}C_6)$Alkyl, $(C_1\text{-}C_6)$Alkoxycarbonyl, wobei die fünf letzt genannten Reste halogeniert sein können, und
m,n = unabhängig voneinander eine Zahl von 1 bis 4 bedeuten.

3. Verfahren gemäß Ansprüchen 1 oder 2, wobei in Formel I
A = N
Z = O oder NH,
R = H oder halogeniertes $(C_1\text{-}C_4)$Alkyl
$R^1$ = H, Halogen, Amino, Hydroxy oder

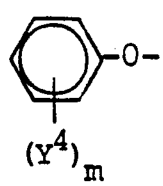

$Y^4$ = H, Cl, $CF_3$, $NH_2$, -NCO oder $(C_1\text{-}C_2)$Alkoxycarbonyl, die in Position 2, 4 oder 6 des Phenoxyrestes orientiert sind, und
m,n = eine Zahl von 1 bis 3 bedueten.
bedeuten.

4. Mittel, enthaltend eine Verbindung der Formel I von Ansprüchen 1, 2 oder 3.

5. Verwendung von Verbindungen der Formel I von Ansprüchen 1, 2 oder 3 zur Herstellung von Pflanzenschutzmitteln.

Patentansprüche für folgenden Vertragsstaat: Spanien

1. Verfahren zur Herstellung der Verbindungen der Formel I

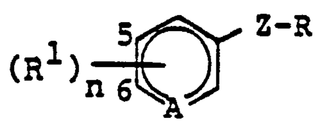

(I),

worin

A = N oder N→O

Z = O oder NH,

R = H, halogeniertes ($C_1$-$C_8$)Alkyl, halogeniertes ($C_2$-$C_8$)Alkenyl oder halogeniertes ($C_2$-$C_6$)Alkinyl, sowie für Z = O ($C_1$-$C_4$)Alkoxycarbonyl

$R^1$ = bei gleicher oder verschiedener Bedeutung Wasserstoff, Halogen, Amino, -NHOH Hydroxy, Phenylazo, das im Phenylring ein-oder mehrfach durch Halogen, Nitro, Carboxy, Sulfo,($C_1$-$C_8$)-Alkyl, ($C_1$-$C_8$)-Alkoxy, halogeniertes ($C_1$-$C_8$)Alkyl oder halogeniertes ($C_1$-$C_8$)Alkoxy substituiert sein kann,

einen Rest der Formeln

Y = C = N-, $Y^1Y^2$C = N-, $Y^3$NH-oder

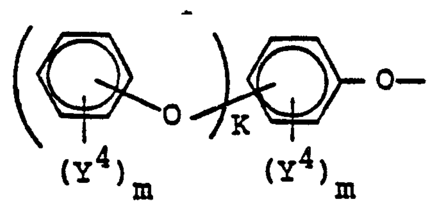

Y = O oder S

$Y^1$ = Halogen, ($C_1$-$C_3$)Alkylthio, ($C_1$-$C_3$)Alkylsulfenyl, ($C_1$-$C_3$)Alkylsulfonyl,

$Y^2$ = einen für $Y^1$ genannten Rest oder Mercapto,

$Y^3$ = ($C_1$-$C_6$)Alkyl, ($C_1$-$C_6$)Alkylsulfenyl, die beide halogeniert sein können, Phenylsulfenyl, das durch ($C_1$-$C_6$)-Alkyl, ($C_1$-$C_6$)Alkoy, ($C_1$-$C_6$)Alkoxy-carbonyl, Halo($C_1$-$C_6$)alkyl, Halo($C_1$-$C_6$)alkoxy, Halo($C_1$-$C_6$alkyl)carbonyl, Halogen, Nitro oder Cyano substi tuiert sein kann, Phosphoryl oder Thiophosphoryl, die beide durch zwei Reste der Gruppe ($C_1$-$C_8$)Alkoxy, ($C_1$-$C_6$)Alkylthio, Amino, ($C_1$-$C_6$)Alkylamino oder Di($C_1$-$C_6$alkyl)amino substituiert sein können,

$Y^4$ = unabhängig voneinander H, Halogen, $NO_2$, $NH_2$, -$NHY^3$, -NHOH, -NCO, NCS, OH, ($C_1$-$C_6$)Alkyl, ($C_2$-$C_6$)-Alkenyl, ($C_2$-$C_6$)Alkinyl, ($C_1$-$C_6$)Alkoxy, ($C_1$-$C_6$)Alkoxy-carbonyl, wobei die fünf letzt genannten Reste halogeniert sein können, Nitro, Cyano, Carboxy oder ein Carboxylat-Salz,

K = 0 oder 1,

m,n = unabhängig voneinander eine Zahl von 1 bis 4 bedeuten,

dadurch gekennzeichnet, daß man

a) zur Herstellung von Verbindungen mit Z = O und R≠H eine Verbindung der Formel (II)

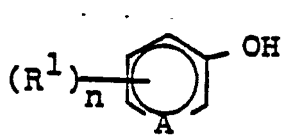

(II)

$a_1$) mit einer Verbindung der Formel $Y^1$-R', worin R' die Bedeutung von R außer H und $Y^1$ einen nucleofugen Rest wie Halogen, Phenylsulfonyl, Phenylsulfinyl, Tosyl oder Mesyl bedeutet, in Gegenwart einer Base oder

$a_2$) mit einem halogenierten ($C_2$-$C_8$)Alken oder halogeniertem ($C_2$-$C_8$)Alkin in Gegenwart einer Base, oder

$a_3$) einem halogiertem Carben der Formel C$(Hal)_2$), wobei Hal bei gleicher oder verschiedener Bedeutung Halogen, insbesondere F oder Cl bedeutet, oder

$a_4$) mit einer Verbindung der Formel $Y^1$-CO-$R^2$, worin $R^2$ = Halogen, halogeniertes ($C_1$-$C_7$)Alkyl, halogeniertes ($C_2$-$C_7$)Alkenyl oder halogeniertes ($C_2$-$C_7$)Alkinyl bedeutet, umsetzt und das erhaltene Zwischenprodukt der Formel (III)

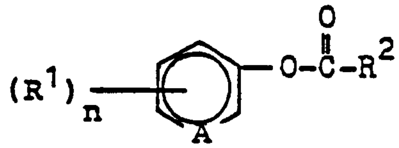

(III)

halogeniert, oder

$a_5$) zur Herstellung von Verbindungen mit Z=O und R= perhalogeniertes ($C_1$-$C_8$)Alkyl eine Verbindung der Formel (II) mit einem perhalogenierten ($C_1$-$C_8$)Alkan in Gegenwart einer Base oder in Gegenwart von HF, oder

b) zur Herstellung von Verbindungen der Formel I mit Z=O und R≠H eine Verbindung der Formel - (IV)

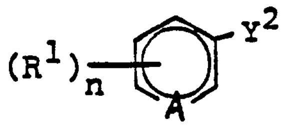

(IV),

worin $Y^2$ eine Abgangsgruppe wie Halogen, Nitro, Diazonium, Alkylsulfonyl, halogeniertes Alkylsulfonyl, Phenylsulfonyl, das im Phenylrest beispielsweise durch Alkyl, Halogen substituiert sein kann, bedeutet, mit einem Alkalisalz einer Verbindung der Formel $OH-CH_2-R^3$, worin $R^3$ die Bedeutung von $R^2$ außer Halogen besitzt, umsetzt oder

c) zur Herstellung von Verbindungen der Formel I mit Z=O und R=H eine Verbindung der Formel - (V),

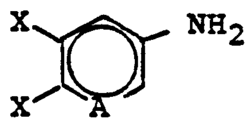

wobei X= unabhängig voneinander Halogen bedeutet, diazotiert und hydrolysiert oder

d) zur Herstellung von Verbindungen mit Z-R = $NH_2$ eine Verbindung der Formel (VI)

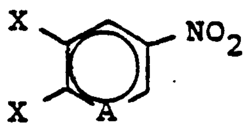

(VI)

reduziert, oder

e) zur Herstellung von Verbindungen der Formel I, die in Position 6 einen, gegebenenfalls wie oben angegeben substituierten Phenylazo-Rest enthalten, eine Verbindung der Formel (VII)

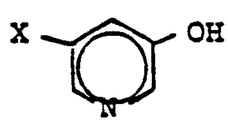

(VII)

mit einem Phenyldiazonium-Ion, das, wie für Phenylazo von Formel (I) angegeben, substituiert sein kann, umsetzt, oder

f) zur Herstellung von Verbindungen mit

$R^1$ =

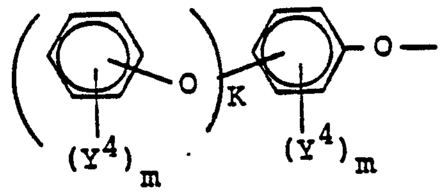

eine Verbindung der Formel (VIII)

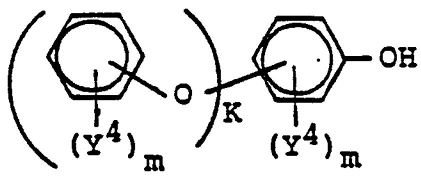

(VIII)

mit einer Verbindung der Formel IX

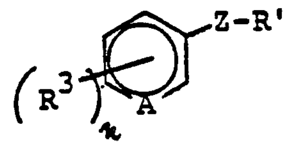

worin $R^3$ eine nucleofuge Abgangsgruppe wie Halogen, Alkylsulfinyl, Phenylsulfinyl, Phenylsulfonyl oder Alkylsulfonyl bedeutet, und R′ die Bedeutung von R außer Wasserstoff besitzt, umsetzt,
und die unter a) bis f) erhaltenen Produkte gegebenenfalls derivatisiert.

2. Verfahren gemäß Anspruch 1, wobei in Formel I
A = N oder N→O
Z = O oder NH,
R = H, halogeniertes $(C_1-C_8)$Alkyl,
$R^1$ bei gleicher oder verschiedener Bedeutung Wasserstoff, Halogen, Amino, Hydroxy, Phenylazo, das im Phenylring ein-oder mehrfach durch Halogen, Nitro oder Carboxy substituiert sein kann,
einen Rest der Formeln
$Y=C=N-$, $Y^1Y^2C=N$-oder

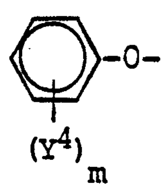

,

Y = O oder S
$Y^1$ = Halogen oder $(C_1-C_3)$Alkylthio,
$Y^2$ = einen für $Y^1$ genannten Rest oder Mercapto,
$Y^4$ = unabhängig voneinander H, $NO_2$, $NH_2$, -NHOH, Halogen, -NCO, NCS, OH, $(C_1-C_6)$Alkyl, $(C_1-C_6)$Alkoxycarbonyl, wobei die fünf letzt genannten Reste halogeniert sein können, und
m,n = unabhängig voneinander eine Zahl von 1 bis 4 bedeuten.

3. Verfahren gemäß Ansprüchen 1 oder 2, wobei in Formel I
A = N
Z = O oder NH,
R = H oder halogeniertes $(C_1-C_4)$Alkyl
$R^1$ = H, Halogen, Amino, Hydroxy oder

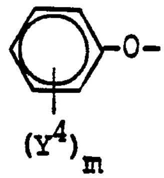

$Y^4$ = H, Cl, $CF_3$, $NH_2$, -NCO oder ($C_1$-$C_2$)Alkoxycarbonyl, die in Position 2, 4 oder 6 des Phenoxyrestes orientiert sind, und m,n = eine Zahl von 1 bis 3 bedeuten.
bedeuten.